(19) Europäisches Patentamt
European Patent Office
Office européen des brevets

(11) Publication number : **0 466 315 A2**

(12) # EUROPEAN PATENT APPLICATION

(21) Application number : **91304839.3**

(22) Date of filing : **29.05.91**

(51) Int. Cl.$^5$ : **A61K 31/725,** A61K 31/56

(30) Priority : **30.05.90 US 530628**

(43) Date of publication of application :
**15.01.92 Bulletin 92/03**

(84) Designated Contracting States :
**AT BE CH DE DK ES FR GB GR IT LI LU NL SE**

(71) Applicant : **GILLESPIE, Larrian**
**120 South Spalding Drive, Suite 210**
**Beverley Hills, CA 90212 (US)**

(72) Inventor : **GILLESPIE, Larrian**
**120 South Spalding Drive, Suite 210**
**Beverley Hills, CA 90212 (US)**

(74) Representative : **Lord, Hilton David et al**
**Marks & Clerk 57-60 Lincoln's Inn Fields**
**London WC2A 3LS (GB)**

(54) **Compositions containing xylan sulphates for affecting growth factor function and for inhibiting fibroblast proliferation.**

(57) The present invention relates to compositions, methods, and processes for affecting the function or activity of growth factors, and for treating improper production of, or improper physiological responses to, growth factors, and for inhibiting fibroblast proliferation, the compositions comprising xylan sulphates, optionally together with steroids and growth factors.

EP 0 466 315 A2

The present invention pertains to xylan sulfates, and fractions thereof, alone or in combination with steroids, for affecting the function of growth factors, and for treating improper supplies of, or improper physiological responses to, growth factors, and for inhibiting fibroblast proliferation. Encompassed within this invention are compositions, processes and methods having clinical, experimental, pharmaceutical, and therapeutic applications.

There exist, in organisms, many proteins that are released from one cell type, but exert dramatic effects on a variety of other cells, either neighboring cells or distant cells. One class of such polypeptides are the growth factors; these are found in a variety of tissues, both normal and neoplastic.

For instance, various endothelial cell growth factors (ECGF's) have been isolated from such tissues as the retina, cartilage, and hypothalamus. One such ECGF, basic fibroblast growth factor (bFGF), has been isolated from sources such as brain, pituitary, hypothalamus, eye, adrenal gland, chondrosarcoma, and hepatoma cells; acidic fibroblast growth factor (aFGF) has also been found in neural tissue.

As additional examples, transforming growth factor-Beta (TGF-Beta) is found in tumors, and in normal tissue cells such as placenta, kidneys, and platelets; transforming growth factor-Alpha (TGF-Alpha) is synthesized by transformed cells. Tumor necrosis factor-Alpha (TNF-Alpha) can be isolated from the serum of mice primed with bacillus Calmette-Guerin (BCG), and treated with endotoxin. Platelet derived growth factor (PDGF) is released from platelets when they adhere to the surface of an injured blood vessel, and is also found in bone; in addition, endothelial cells that line the inner surface of blood vessels produce PDGF under the tight control of a number of factors that regulate transcription of PDGF genes.

Many physiological mechanisms have been identified as being caused by, or affected by, specific growth factors. aFGF and bFGF are potent mitogens <u>in vitro</u> for endothelium. PDGF is a mitogen for various types of mesenchymal cells, and stimulates vasoconstriction, chemotaxis, activation of glycogen synthetase, and other processes; experimentation <u>in vitro</u> suggests that pulsile delivery of PDGF is required to avoid cataractogenesis. TNF effects enhancement of fibroblast growth, stimulation of collagenase release from several mesenchymal cell types, and bone resorption. TGF- Alpha and EGF induce mitosis and migration of human skin keratinocytes, with the former appearing to be the more potent of the two in this application. TGF-Beta inhibits proliferation of primary or secondary cultures of epithelial cells; administered subcutaneously in wound chambers or by injection, TGF-Beta also rapidly induces fibrosis.

A particular physiological mechanism which appears to be susceptible to a significant number of growth factors is angiogenesis, i.e., the formation of new blood vessels; aFGF, bFGF, angiogenic, TGF-Alpha, TGF-Beta, TNF, and the prostaglandins, particularly prostaglandin $E_1$, ($PGE_1$), and prostaglandin $E_2$, ($PGE_2$,), have all been found to be angiogenic in particular environments. For instance, TGF-Alpha, TGF-Beta, and EGF are all known to induce angiogenesis <u>in vivo</u>; bFGF and aFGF cause angiogenesis in chick chorioallantoic membrane (CAM), in the cornea, and in wound-healing models.

Growth factors have been implicated in various pathological conditions. PDGF stimulates the proliferation of vascular smooth muscle cells, which can cause pathological narrowing of the lumen of a blood vessel at the site of an atherosclerotic plaque; PDGF may further have a role in fibroproliferative pathological processes in addition to atherosclerosis, including pulmonary fibrosis, glomerulonephritis, myelofibrosis, keloid formation, and carcinogenesis. TGF-Alpha can induce such signs of psoriasis as angiogenesis and abnormal proliferation of epidermal cells; excess amounts of both messenger RNA molecules for TGF-Alpha, and TGF-Alpha itself, have been detected in psoriatic lesions.

Further as to this point, it appears that failure to produce inhibitory growth factors, or loss of cellular response thereto, may have an oncogenic effect on cells. For instance, while TGF-Alpha is known as a cell growth stimulator, TGF-Beta has been discovered to inhibit cell proliferation; the absence or deficiency of TGF-Beta, or of the negative growth influence exerted thereby, appears to play a role in the development of breast cancer.

Angiogenesis, previously discussed, is known to take place in connection with both normal and pathological physiological activities. For instance, angiogenesis occurs in embryo development, corpus luteum formation, and wound healing; conditions characterized by deleterious or excessive angiogenesis include chronic inflammation, certain immune responses, and tumor growth.

Persistent angiogenesis appears to be essential to tumor growth, as well as the dominant pathology in a variety of nonneoplastic diseases. Such diseases which have been thus identified include diabetic retinopathy, retrolental fibroplasia, and neovascular glaucoma; in fact, abnormal neovascularization is one of the most common causes of blindness worldwide. Others are rheumatoid arthritis, hemangiomas, angiofibromas, and psoriasis (previously discussed). These pathologic states, previously thought to be unrelated, can be characterized as "angiogenic diseases".

There are also pathological conditions characterized by inadequate angiogenesis. These include delayed wound healing, nonhealing fractures, and certain congenital malformations, such as hemifacial microsomia in which local or regional vascularization failed during fetal growth.

As previously discussed, a variety of growth factors has been found to induce angiogenesis. With angiogenesis having been thus identified in connection with various diseases, it appears that the activity or presence of growth factors, or the lack thereof, would likewise be implicated with such diseases.

It has also been discussed that growth factors can be employed to affect tissues beneficially. EGF and TGF-Beta have both been found to accelerate wound healing; specifically, TGF-Beta has a number of specific biochemical actions that foster the formation of collagen and connective tissue during inflammation and wound healing. Both aFGF and bFGF, discussed earlier as being potent mitogens in vitro for endothelium, are also known to repair endothelium in vivo.

Several conclusions are suggested by the foregoing.

The evidence is compelling that different diseases can be caused by a variety of conditions pertaining to growth factor presence or activity, including the following:

-- excessive or insufficient tissue response to growth factor presence;

-- excessive or insufficient growth factor activity; and

-- an excess or insufficiency of one or more growth factors themselves.

Accordingly, it would appear that means for affecting these conditions would be useful both in vivo and in vitro, in therapeutic, pharmaceutical, experimental and clinical applications.

For instance, a disease resulting from a deficiency in the production of a growth factor, or from a deficiency in response to a growth factor, may be susceptible to an agent which either stimulates the activity of whatever amount of growth factor is present (such as by chemical interaction therewith), or stimulates the response of tissue thereto. Types of diseases responding to this type of treatment may include those resulting from the failure of growth inhibition provided by particular growth factors.

One possibility, as earlier discussed, is breast cancer caused by a deficiency in tissue response to TGF-Beta. Such breast cancer development may respond favorably to an agent providing growth factor stimulation.

Conversely, where the problem is an excess, rather than a deficiency, of growth factor or response thereto, the solution may be treatment with an agent which inhibits instead of stimulating growth factor activity or function. For instance, local or systemic administration of just such an agent may be indicated for the angiogenic diseases, previously discussed.

Agents providing stimulatory inhibitory effects would also be useful for laboratory procedures, e.g., to stimulate and inhibit growth factor response in cultured tissue, or in other media.

Agents having a particular effect on the function or activity of growth factors already present in tissues would logically affect growth factors in the same manner when used in concert therewith for application to tissues. Like the agent alone, the combination of agent and growth factor would also be expected to have utility in therapeutic, pharmaceutical, experimental and clinical applications.

Specifically, such agents would appear to be particularly suited for use in concert with growth factors in pharmaceutical applications, especially for the purpose of stimulating the function or activity of such growth factors. For example, with respect to growth factors which accelerate wound and fracture healing, or which increase neovascularization in the ischemic or infarcted heart, suitable agents could be administered in concert therewith.

A point to be considered with respect to these proposed applications is that tissue response to growth factors is not readily predictable. For instance, TNF promotes both necrotic (e.g., hemorrhagic necrosis in transplanted tumors, killing transformed cell lines) and growth (e.g., angiogenesis) responses in tissue. TGF-Beta is particularly noted for paradoxical behavior, with the following effects having been observed: promotion of terminal squamous differentiation of bronchial epithelial cells, but inhibition of B-cell maturation short of the mature immunoglobin-secreting state; potentiation of the steroidogenic effect of FSH on ovarian granulosa cells, contrasted with suppression of the steroidogenic effect of ACTH on adrenal cells; stimulation by human TGF-Beta of collagen and fibronectin synthesis by fibroblasts, as opposed to the failure of this growth factor to stimulate collagen synthesis by bone cells; stimulation of cells of mesenchymal origin, but inhibition of cells of epithelial or neuroectodermal origin ("Human Transforming Growth Factor-Beta (hTGF-Beta)", Collaborative Research Incorporated, Two Oak Park, Bedford, MA 01730, 1988; "TGF-Beta: Beta 2, Porcine"), R & D Systems, Inc., 614 McKinley Place, N.E., Minneapolis, MN 55413; Beta 1 Human and Porcine"); inhibition of aortic endothial cells in vitro, in contrast to angiogenic activity in vitro ("Angiogenesis-Mechanisms and Pathobiology", KLAGSBRUN et al., Current Communications in Molecular Biology, Cold Spring Harbor Laboratory/1987).

The result of interaction between particular tissues and growth factors can depend on a multlplicity of factors, not all of which may be evident. For instance, as to the growth factor TGF-Beta, its activities depend, inter alia, on the following: tissue origin of the target cells; whether the target cells are normal or neoplastic; the state of differentiation of the target cells; the presence and activity of other growth factors; and (for in vivo applications) the type of culture method. Other considerations may be relevant for this and other growth factors.

Moreover, particular pathological conditions have been treated by affecting angiogenesis.

Inhibition of angiogenesis has been found to halt, and even cause the regression of, tumor growth ("Angiogenesis Inhibition and Tumor Regression Caused by Heparin or a Heparin Fragment in the Presence of Cortisone", FOLKMAN et al., Science, Vol. 221, 1983, pp. 719-725") and to prevent tumor metastasis (FOLKMAN et al., European Pat. No. 0 114 589, published August 1, 1984). It is further known that pentosan polysulfate (GILLESPIE et al., U.S. Patent No. 4,820,693, issued April 11, 1989), also known as xylan sulfate, and heparin (the previously cited FOLKMAN et al. article and European patent), in combination with particular steroids, inhibit angiogenesis.

However, there is no such disclosure or suggestion that the activity of the xylan sulfate-steroid combination depends on several factors, including the identity of the particular growth factor and steroids.

Accordingly, although, as indicated, it is known that xylan sulfate and steroid act in concert to inhibit angiogenesis, there is no disclosure or suggestion in the art that, for particular steroids and growth factors, the combination of xylan sulfate and steroid will instead stimulate rather than inhibit the function of the growth factor, and that, where the function of the particular growth factor is angiogenic, such stimulation will be in the form of stimulating rather than inhibiting angiogenesis.

It is further not known or suggested in the art that the combination of xylan sulfate and steroid has utility for treating improper growth factor supplies in the tissues, or improper physiological responses to growth factors.

Further as to what is known in the art, the previously-cited FOLKMAN et al. article and European patent disclose that heparin potentiates tumor-induced angiogenesis. The literature further discloses that heparin and xylan sulfate both stimulate the activity of aFGF, but not bFGF ("Modulation by Heparins of Pentosan Polysulfate of the Effects of Acidic and Basic Human Fibroblast Growth Factors (aFGF and bFGF) on the Repair of a Mechanical Lesion of the Endothelium", KLEIN-SOYER et al., Abstracts-Xth International Congress on Thrombosis, the Mediterranean League against Thromboembolic Diseases, C.D. Michalopoulos, M.D. (Ed.), Athens, Greece, May 22-27, 1988); this distract even suggests a drug combining fibroblast growth factors with heparin, low molecular weight heparin, or xylan sulfate to stimulate the repair of injured endothelium, and prevent blood vessel wall interactions leading to thrombosis or atherosclerosis.

However, notwithstanding such disclosure pertaining to xylan sulfate for stimulating the activity of a particular growth factor, i.e., Acidic or Basic Human Fibroblast Growth Factor, there is no disclosure or suggestion in the art that xylan sulfate may instead inhibit growth factor activity. Further with respect to fibroblasts, heparin has been shown to inhibit human scleral fibroblast proliferation ("Inhibition of Human Scleral Fibroblast Proliferation with Heparin", DEL VECCHIO et al., Investigative Ophthalmology & Visual Science, Vo. 29, No. 8, pp. 1272-1274 (Aug. 1988); however, there is no disclosure or suggestion in the art that xylan sulfate has utility for inhibiting the proliferation of fibroblasts, such as human scleral fibroblasts.

There is also no disclosure or suggestion in the art that particular molecular weight fractions or ranges of xylan sulfate affect growth factor function, either alone or in combination with steroids. Yet further, there is no disclosure or suggestion in the art that xylan sulfates derived from different sources can differ in composition or molecular weight, or that such xylan sulfates, or compositions differing only in the source of their xylan sulfate components, can differ in their activities.

## SUMMARY OF THE INVENTION

It has been discovered that xylan sulfate fractions, separated from xylan sulfate according to molecular weight or range thereof, alone or in combination with steroids, can be used to affect growth factor function. This discovery can be employed both in vivo and in vitro, in a variety of compositions, processes, and methods.

For example, in the laboratory, compositions of the invention may be applied to cultured tissue, or other substrates, to induce or affect physiological activity. The invention will also have utility in therapeutic and pharmaceutical applications.

Accordingly, included within the scope of the invention are compositions for affecting the function or activity of a growth factor. These compositions comprise polysaccharides consisting essentially of specific weight average molecular weight fractions of xylan sulfate.

Included are compositions for which the effect on growth factor function or activity is stimulation thereof; also included are compositions which, in contrast, inhibit growth factor function or activity. The growth factors may be any whose activity is thusly affected; particular growth factors include ECGS, aFGF, bFGF, PDGF, TGF-Beta, EGF, and TNF-Alpha.

Similarly, any xylan sulfate fraction of a specific molecular weight or molecular weight range which will affect growth factor function is within the scope of the invention. Particular such fractions are not limited to, but include,those having weight average molecular weights of 8820, 5290, 4370, 3900, and 4210.

Such growth factor function or activity-affecting compositions may further include one or more steroids.

Possible steroids include cortisone, hydrocortisone, 11 alpha epihydrocortisol, tetrahydro S, 17 alpha hydroxy progesterone, deltacortisone, and cortexolone. It is emphasized that this group of steroids is provided only as a representative list of suitable steroids; any other steroids which, in concert with the above-indicated components, will provide the requisite effect to growth factor function or activity, are also suitable.

The invention is also directed to processes for thusly affecting growth factor function or activity, by treating one or more growth factors with such xylan sulfate fractions and steroids, as discussed above. Further, pharmaceutically effective amounts of these substances may be employed as compositions of the invention, and may be administered to treat an improper tissue production of, or an improper physiological response to, a growth factor. Such treatments may be utilized both for excesses of, or excessive responses to, growth factors, as well as for insufficiencies of, or insufficient responses to, growth factors.

The invention is further directed to compositions, for inhibiting growth factor function, which comprise xylan sulfate, and are essentially free of steroids. Such compositions may be used to treat growth factors for the indicated purpose of inhibiting growth factor function.

In pharmaceutical applications, such compositions may include pharmaceutically effective amounts of xylan sulfate, while still being essentially free of steroids. Accordingly, compositions comprising pharmaceutically effective amounts of xylan sulfate, and being essentially free of steroids, may be administered to treat a tissue excess of, or an excessive physiological responsiveness to, growth factors.

Also within the scope of the invention are compositions, comprising xylan sulfate, for inhibiting fibroblast proliferation. For pharmaceutical applications, such compositions may comprise pharmaceutically effective amounts of xylan sulfate.

Yet further within the scope of the invention are compositions, comprising pharmaceutically effective amounts of xylan sulfate and steroids, for treating an improper tissue production of, or an improper physiological response to, growth factors. Further included are methods, comprising pharmaceutical administration of such compositions, for the same purposes.

As previously discussed with respect to the compositions of the invention utilizing xylan sulfate fractions, the growth factors include all those for which function or activity may be affected by such compositions; specific such growth factors include ECGS, aFGF, bFGF, PDGF, TFG-Beta, EGF, and TNF-Alpha. The steroids which may be any used in concert with xylan sulfate are all those suitable for use in concert with xylan fractions, as previously discussed. Particular examples of such steroids include cortisone, hydrocortisone, 11 alpha epihydrocortisol, tetrahydro S, 17 alpha hydroxy progesterone, deltacortisone, and cortexolone.

Also included within the scope of the invention are pharmaceutically effective amounts of xylan sulfate and steroids, for the purpose of stimulating angiogenesis. Any steroid which, used in concert with xylan sulfate, will stimulate angiogenesis is suitable; representative samples of such steroids include cortisone, hydrocortisone, 11 alpha epihydrocortisol, tetrahydro S, 17 alpha hydroxy progesterone, deltacortisone, and cortexolone.

The xylan sulfate of the invention maybe derived from any suitable source. Two xylan sulfates which may be used are those derived from beechwood and from larch.

Figure 1 is a graph showing the effect of different concentrations of triamcinolone on in vitro proliferation of human scleral fibroblasts from different patients.

Figure 2 is a graph showing the effect of different concentrations of $SP_{54}$ xylan sulfate on in vitro proliferation of human scleral fibroblasts from different patients.

Figure 3 is a comparative graph, concurrently depicting the effects of triamcinolone and $SP_{54}$ xylan sulfate or in vitro proliferation of human scleral fibroblasts from different patients.

Figure 4 shows ultraviolet spectra for $SP_{54}$ xylan sulfate, Prednisone, and a Prednisone/$SP_{54}$ xylan sulfate mixture.

Figure 5 shows ultraviolet spectra for 0.33% larch xylan sulfate II in aqueous pH 5.8, pH 12.2, and pH 1.5 solutions.

Figure 6 shows the infrared spectrum for $SP_{54}$ xylan sulfate.

Figure 7 shows the infrared spectrum for larch xylan sulfate I.

Figure 8 shows that the infrared spectrum for larch xylan sulfate II.

Figure 9 shows the infrared spectrum for larch xylan.

Figure 10 shows the proton nuclear magnetic resonance spectrum of $SP_{54}$ xylan sulfate.

Figure 11 shows the carbon thirteen nuclear magnetic resonance spectrum of $SP_{54}$ xylan sulfate.

Figure 12 shows the proton nuclear magnetic resonance spectrum of larch xylan sulfate II.

Figure 13 snows the carbon thirteen nuclear magnetic resonance spectrum for larch xylan sulfate II.

Figure 14 shows the high pressure liquid chromatograph of $SP_{54}$ xylan sulfate in stock solution form.

Figure 15 shows the high pressure liquid chromatograph of $SP_{54}$ xylan sulfate in powder form.

Figure 16 shows the high pressure liquid chromatograph of larch xylan sulfate I.

Figure 17 shows the high pressure liquid chromatograph of larch xylan sulfate II.

Figure 18 is a graph showing an elution profile of the xylan sulfate $SP_{54}$.

Figure 19 is a graph displaying the presence of five fractions in the xylan sulfate $SP_{54}$.

Figure 20 is a graph showing the elution profiles of the five fractions distinguished in the xylan sulfate $SP_{54}$.

Figure 21 is a graph showing the reading of the five $SP_{54}$ xylan sulfate fractions at 490 nanometers.

Figure 22 and 23 are graphs comparing the effect of various compositions on endothelial cell growth.

## DESCRIPTION OF THE PREFERRED EMBODIMENTS

The terms"activity" and "function" are used interchangeably herein, with respect to growth factors.

The xylan sultates suitable for the compositions, processes and methods of the invention include those which, in the absence of steroids, inhibit growth factor function in vivo or in vitro, or otherwise in any experimental, clinical, therapeutic, or pharmaceutical application. Also suitable are those which, in combination with one or more steroids, more broadly affect, i.e., either inhibit or stimulate, growth factor function, also in vivo or in vitro, as well as in any experimental, clinical, therapeutic or pharmaceutical application.

The basic structure of xylan sulfates is understood to be a beta (1->4) linked chain of D-xylose units. However, it appears, from the present state of the art for analytically defining xylan sulfates, that compositions and molecular weights vary for particular xylan sulfates, according to the source from which they are obtained, or the method by which they are extracted or synthesized. Composition and molecular weight variations may even be present in a particular xylan sulfate obtained, extracted, or synthesized from the same source or process.

Different researchers have obtained different results in attempting to determine the structure of xylan sulfates. However, the general consensus is that they have a random structural formula of

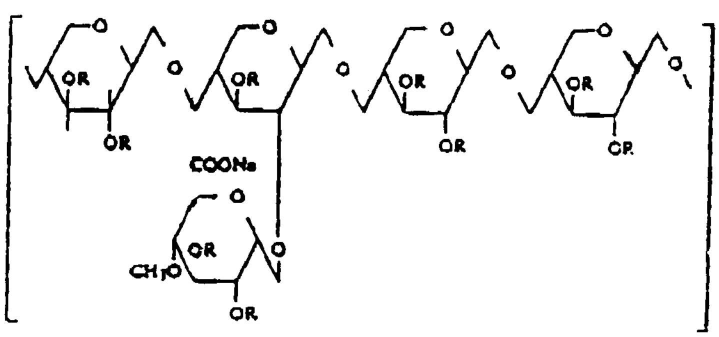

wherein R is at least one member selected from the group consisting of H and $-SO_3Y$, and Y is at least one member selected from the group consisting of H and a pharmaceutically acceptable cation. Sodium is the preferred such cation; potassium and magnesium are also suitable, as is the ammonium radical.

Xylan sulfates may be obtained from such sources as beechwood, oat spelts, and larch. Suitable xylan sulfates are disclosed in the following: U. S. Patent No. 4,820,693, issued April 11, 1989, in the name of GILLESPIE; U.S. Patent No. 4,699,900, issued October 13, 1987, in the name of BAYOL et al.; U.S. Patent No. 4,713,373, issued December 15, 1987, also in the name of BAYOL et al.; U.S. Patent No. 2,689,848, issued September 21, 1954, in the name of HUSEMANN et al.; and "Inhibition of SMC Proliferation", Vol. 46, No. 6, p. 798. The entire texts of these materials are incorporated herein by reference thereto.

One commercially available form of xylan sulfate which may be used is $SP_{54}$, sold (both as a powder and in stock solutions) by BENE ARZNEIMITTEL GMBH, 8,000 Munich 71, Federal Republic of Germany; $SP_{54}$ xylan sulfate is obtained from Fagus sylvatica, a type of beechwood. Another is Thromboacid R , $\overline{M}_w$ about 4,000.

As previously noted, a particular xylan sulfate may be comprised of chains of varying length, i.e., molecular weight. Also suitable for the invention are xylan sulfate fractions of any molecular weight, or molecular weight range, which, alone or in combination with one or more steroids, affect growth factor function as previously described with respect to the combination of xylan and steroid.

Such xylan sulfates of particular molecular weight or molecular weight range may be obtained by fractionation of a xylan sulfate comprised of different molecular weight chains. These fractions may be obtained in the form of fractions of specific weight average molecular weight, and by any appropriate separation technique, such as gel chromatography.

Examples of xylan sulfate fractions having utility in this invention are those characterized by the following weight average molecular weights: 3,900; 4,210; 4,370; 5,290; and 8,820. However, the invention is not limited to these particular fractions; any molecular weight or molecular weight range xylan sulfate fraction which, alone

or in combination with one or more steroids, affects the function of one or more growth factors, is also encompassed within this invention.

As previously discussed, compositions and molecular weights of xylan sulfates can vary according to their source, and according to the method by which they are extracted or synthesized; also, xylan sulfate fractions of different molecular weights or molecular weight ranges may be obtained from a particular xylan sulfate. Procedures subsequently discussed herein confirm composition and molecular weight distinctions between xylan sulfates from different sources, as well as differences in activity between xylan sulfates derived from different sources, compositions differing in the source of their xylan sulfate components, and between xylan sulfate fractions of different weight average molecular weights.

The steroids of the invention are any of those which, in combination with one or more xylan sulfates, or in combination with one or more xylan sulfate fractions, or in combination with any combination of such xylan sulfates and xylan sulfate fractions, affects the function of one or more growth factors, is within the scope of this invention. Eligible steroids include the "angiostatic" steroids, as defined in U.S. Patent No. 4,820,693, previously discussed, as well as the particular steroids disclosed therein as having utility in combination with pentosan polysulfate. The steroids disclosed in U.S. Patent No. 4,771,042, issued September 13, 1988, in the names of BRAUGHLER et al., the entire text of which is incorporated herein by reference thereto, may also be used. Also suitable are the "angiostatic" and "angiotropic" steroids as defined and discussed in the following materials, the entire texts of which are incorporated herein by reference thereto: "A New Class of Steroids Inhibits Angiogenesis in the Presence of Heparin or a Heparin Fragment", CRUM et al., Science, Vol. 30, 1988, pp. 1375-1378; and "Anti-Angiogenesis by Steroids without Glucocorticoid or Mineralocorticoid Activity in the Presence of Heparin", CRUM et al., Journal of Cell Biology, October, 1984, Abstract No. 581, page 158a.

Steroids which may be used include cortisone, hydrocortisone, 11 alpha epihydrocortisol, tetrahydro S, 17 alpha hydroxy progesterone, deltacortisone, and cortexolone. However, these are listed only as representative examples, with the understanding that the invention is not limited to such steroids, but rather, includes all steroids which, as discussed, act in concert with xylan sulfates and fractions thereof to affect growth factor function.

The steroids of the invention as discussed herein are understood to include the salts thereof. In this context, reference to cortisone includes cortisone acetate, reference to hydrocortisone includes hydrocortisone 21-phosphate, etc. For the embodiment of this invention having pharmaceutical applications, such salts are understood to be the physiologically acceptable salts, particularly those which are water-soluble.

The growth factors of the invention include all those suitable for use in concert with xylan sulfates, fractions thereof, and combinations of these with the steroids, as previously discussed. Representative examples of such growth factors are aFGF, bFGF, PDGF, TGF-Beta, TNF-Alpha, EGF, and endothelial cell growth stimulator (ECGS). As with the steroids, discussed above, it is understood that the invention is not limited to these representative examples, but includes all other growth factors suitable for use in concert with xylan sulfates, factions thereof, and combinations of these with steroids.

The xylan sulfates, specific molecular weights and molecular weight fractions thereof, and combinations of such xylan sulfates and fractions with steroids, have utility for in vivo and in vitro applications, including applications pertaining to affecting growth factor response, and including experimental, clinical, pharmaceutical, and therapeutic applications. Such applications can include the use of growth factors in concert with these xylan sulfates, fractions thereof, and combinations of xylan sulfates and xylan sulfate fractions with steroids; these are all hereinafter embraced in the term "compositions of the invention", and the indicated xylan sulfates, xylan sulfate fractions, steroids, and growth factors are understood to be components thereof.

It is understood that the therapeutic and pharmaceutical compositions of the invention may include all suitable pharmaceutical excipients, and will include pharmaceutically effective amounts of the active components therein. Such active components include the xylan sulfates, fractions thereof, and steroids which are present therein.

Suitable experimental applications are laboratory procedures involving cell or tissue culture. Particular compositions of the invention can be applied to achieve the desired results.

The compositions of the invention are also appropriate for therapeutic applications. One example is the previously-discussed case of breast cancer responsive to TGF-Beta; specific compositions of the invention which will stimulate the activity of this growth factor, or the response of mammary tissue to the inhibitory affect thereof, can be administered in suitable pharmaceutical compositions or therapeutic procedures.

Another example concerns inhibition of fibroblast proliferation, with a xylan sulfate composition of the invention. A suitable such xylan sulfate is $SP_{54}$ xylan sulfate; however, the invention is not limited to this particular xylan sulfate, but encompasses all xylan sulfates, and fractions thereof, including those as disclosed herein, which will thusly inhibit fibroblast proliferation.

A particular application pertains to treatment of human ocular, or scleral, fibroblasts, especially, as discussed below, in connection with glaucoma filtration surgery; however, similarly as indicated with respect to xylan sulfates, the invention is not so limited, but extends to all manner of fibroblasts, and their proliferation, susceptible to xylan sulfate treatment.

Proliferation of fibroblasts is a serious problem attendant upon ocular trauma and surgical wound healing. A particular procedure wherein fibroblast proliferation causes complications is glaucoma filtration surgery.

For such surgery to be successful, the trabeculectomy site must remain open to allow fluid drainage, and to maintain reduced pressure. However, fibroblast proliferation can result in the formation of scar tissue at the trabeculectomy site, causing failure of the procedure.

The current treatment for scar prevention is intra-ocular injection of 5-fluorouracil. However, this drug has many toxic side effects.

The synthetic steroid triamcinolone has also been tested for prevention of scar formation following glaucoma filtration surgery. Though not widely used, this drug does offer a degree of protection against scarring; however, response to this steroid can vary between patients, with some experiencing very little inhibition, and others even showing stimulation of scar formation.

Moreover, with any of the drugs presently used for this purpose, there is a 25-35% failure rate. Some patients exhibit no response to treatment, and are accordingly afflicted with the formation of scar tissue following fibroplasia.

Xylan sulfate has been found to inhibit fibroblasts more effectively than triamcinolone acetonide. Furthermore, in contrast to the inconsistency of response to this steroid, xylan sulfate has been found to provide inhibition of fibroblasts with very little variation.

The effect of different concentrations of triamcinolone and $SP_{54}$ xylan sulfate was tested in vitro on human scleral fibroblasts from four different patients. The results obtained from administration of triamcinolone are shown in Fig. 1; for $SP_{54}$ xylan sulfate, in Fig. 2. A comparative evaluation of the two is depicted in Fig. 3.

As shown in these three graphs, the triamcinolone responses exhibit greater variation, and in some instances, even stimulation of fibroblast proliferation (i.e., where the inhibition measurement is negative); there is more consistency of response with $SP_{54}$ xylan sulfate, and no instances of stimulation. The comparative depiction of results in Fig 3 clearly demonstrates the superior efficacy and consistency of $SP_{54}$ xylan sulfate.

Of course, the compositions of the invention are not limited to the particular experimental and pharmaceutical applications discussed above. Other examples, both in vitro and in vivo, will be suitable, in accordance with the properties characterizing the particular compositions.

Where the compositions of the invention comprise multiple components, these may be used both separately and mixed together. Moreover, in addition to the components previously discussed, additional substances may be used with the compositions of the invention, for facilitating such compositions' intended functions.

For instance, administration to an acidic environment may adversely affect a composition of the invention if one or more steroids are incorporated therein. In such instances, a buffering agent may also be included, in amounts sufficient to maintain a pH of about 8 or above, for the purpose of preventing steroid deactivation.

The most preferred buffering agent for compositions of the invention is sodium bicarbonate. Other buffering agents, such as magnesium, potassium, or calcium carbonate, are also suitable.

Similarly, dimethyl sulfoxide (DMSO) may also be included, for the purpose of accelerating the rate and degree of penetration of the composition of the invention through treated tissue. DMSO is a known potentiator which functions as a transport agent, facilitating penetration and flow, of the composition of the invention, into individual cells.

Pharmaceutically effective amounts of appropriate carriers, adjuvants, and excipients may be included in vivo applications.

Amounts of the above-indicated materials for administration in vivo or in vitro, including components of the composition of the invention, and relative proportions of such materials, will depend upon a multiplicity of factors. These include type and degree of response intended, particular materials and amounts thereof used, and sensitivity thereto of the treated tissue, and type of tissue treated. Appropriate amounts and proportions can be determined for particular applications, without undue experimentation, by those of ordinary skill in the art.

For in vivo applications in particular, such as pharmaceutical and therapeutic applications, dosages and proportions are a function of the factors generally determining amounts for such administration. These include, but are not limited to, body weight of the subject, sensitivity of the subject to components used, condition being treated, and intended response. For such purposes, administration may be oral or parenteral, including, inter alia, topical application, intravenous, intra-arterial or subcutaneous injection, and including absorption as well as injection and introduction into bodily apertures or orifices.

SULFATION AND CHEMICAL ANALYSIS OF LARCH XYLAN

Two successive procedures were performed with xylan obtained from larch, for the purpose of preparing sulfated xylan compositions of differing molecular weights; as discussed below, the product of the first procedure served as the starting material for the second. The second product was subjected to chemical analysis to confirm that the intended sulfation had been achieved.

1. Sulfation of Xylan

The procedures discussed below are those disclosed in Example 1 of the previously cited HUSEMANN et al., U.S. Patent No. 2,689,848. Specifically, the first procedure is that as set forth at column 3, lines 48-71; the second, as set forth in column 3, line 72-column 4, line 18.

1300 cc. of pyridine was added, drop by drop, to 300 cc. of chlorosulfonic acid, with the solution being constantly stirred, and cooled to about 30°C. (cooling can, in fact be to 30-40°C.). This mixture was then heated to 65°C., and 150 grams of xylan were added thereto; the source of the xylan, obtained from larch, was Chemical Dynamics Corporation, Post Office Box 395, 3001 Hadley Road, South Plainfield, NJ 07080.

While constantly being stirred, the solution, viscous in consistency, was maintained at 80°C. for 2 1/2 hours, then allowed to flow into 4 liters of methanol with simultaneous vigorous agitation. A precipitate of pyridine salt was thus obtained.

The precipitated pyridine salt was separated by filtration, dissolved in 800 cc. of water, then allowed to stand for 12 hours after the addition of 400 cc. of 5% chlorine dioxide solution; after the dissolution, the precipitate may, if necessary, be filtered again before being allowed to stand for the indicated 12 hours. This solution was allowed to flow into a mixture of 4.8 liters methanol and 250-300 cc. of 33% sodium hydroxide, with constant stirring, so that the solution was always alkaline to phenolphthalein.

The solution was then adjusted to pH 6 by addition of 40 cc. of concentrated acetic acid, resulting in a pure white salt. This product, a sulfuric acid ester salt of xylan designated as xylan sulfate I, was isolated by filtration, washed with methanol, acetone, and ether, and dried at 50° C.; the yield was over 300 grams.

200 grams of the xylan sulfate I product were dissolved in 590 cc. of warm water, then heated to 97° C. in a water bath. This solution was agitated with 8.16 cc. of 5N $H_2SO_4$, and 100 cc. of 33% $H_2O_2$ having been preheated to 80° C., being added during the agitation. The solution was maintained at 97° C. for 30 minutes, then rapidly cooled.

75 cc. of 5N NaOH was added to neutralize the solution, turning it light yellow. Decolorization was effected with a few drops of chlorine dioxide solution.

The decolorized solution was dialyzed against distilled water in suitable cellophane tubes until the dialyzate was free of sulfate, i.e., the outer water showed a negative sulfate reaction. The dialyzate, designated as larch xylan sulfate II, was concentrated to 200 cc. under vacuum at 45° C.; a yield of over 70 grams of a low polymerized salt was precipitated in 800 cc. of a 1:1 alcohol acetone mixture, and dried over phosphorous pentoxide in vacuo for 25 hours.

Prior to the indicated concentration under vacuum, the dialyzate can, if necessary, be cleared of slight cloudiness by means of a filtration.

Molecular weight measurements were taken of the larch xylan sulfate I and larch xylan sulfate II, using gel permeation chromatography. Three columns were utilized-two TSK2500PWXZ, and a TSK4000PWXZ. The solvent was 10% acetonitrile in 0.2M NaCl, and measurements were taken against polystyrene sulfonate standards.

Larch xylan sulfate I was determined to have a weight average molecular weight (Mw) of 10,650, and a number average molecular weight (Mw) of 1,825, with Mw/Mn thereby being 5.83. Larch xylan sulfate I is accordingly very polydisperse, comprising polymer chains of a wide range of molecular weights.

Larch xylan sulfate II was determined to have a Mw of 7,090, a Mn of 5,750, and, correspondingly, Mw/Mn of 1.23. Larch xylan sulfate II is accordingly characterized by low polydispersity, i.e., comprising polymer chains of a relatively narrower range of molecular weights.

2. Chemical Analysis to Ascertain Sulfation of Treated Xylan

An amount of the indicated larch xylan sulfate II was placed in aqueous solution, a few drops of which were added to an aqueous solution of toluidine blue; a color shift resulted. Mixture of larch xylan sulfate II in the indicated aqueous solution, with an aqueous solution of cetylpyridium chloride, caused a copious precipitate. These results confirmed sulfation of the larch xylan.

COMPARATIVE ANALYSES OF THE $SP_{54}$ AND LARCH XYLAN SULFATES

Various analytical procedures were conducted with $SP_{54}$, and with the larch xylan sulfates I and II. These procedures, as discussed below, further confirmed that the sulfated larch xylan compositions are in fact xylan sulfates, and that there are differences between these larch xylan sulfates and $SP_{54}$.

2. Ultraviolet and Infrared Spectra of $SP_{54}$ and Larch Xylan Sulfates, and Infrared Spectrum of Larch Xylan.

Ultraviolet spectra were taken of $SP_{54}$ xylan sulfate, of Prednisone, and of a combination of Prednisone and $SP_{54}$ xylan sulfate (the significance of the spectra for the latter two compositions is not discussed further herein). Solutions of each of these three compositions were prepared for subjecting to ultraviolet analysis.

The $SP_{54}$ solution, Solution 1, was prepared by mixing 1.0 ml. of a $SP_{54}$ stock solution, having a concentration of 1.111 mg. ml., with 2.0 ml. water, resulting in a solution b of 0.370 mg./ml. $SP_{54}$. To obtain the Prednisone solution-Solution 2-1.0 ml. of water was added to 2.0 ml. of 0.37 mg./ml. Prednisone. Solution 3 was obtained by combining 1.0 ml. of the $SP_{54}$ stock solution and 2.0 ml. of the indicated Prednisone solution.

The ultraviolet spectrum for $SP_{54}$ xylan sulfate is shown as spectrum A (Prednisone being spectrum B, and the Prednisone-$SP_{54}$ mixture being spectrum C) in Fig. 4. The values for these spectra are tabulated in Table 1 below; the heading "A" of each column refers to absorbance.

TABLE 1

| SOLUTION | A 262 NM | A 250 NM | A 223 NM |
|---|---|---|---|
| 1 | 0.052 | 0.035 | 0.092 |
| SP54 | 0.053 | 0.035 | 0.072 |
| | 0.053 | 0.039 | 0.089 |
| AVERAGE 1 | 0.053 | 0.036 | 0.084 |
| 2 | 0.342 | 0.436 | 0.867 |
| PREDNISONE | 0.341 | 0.436 | 0.857 |
| | 0.358 | 0.466 | 0.898 |
| AVERAGE 2 | 0.347 | 0.446 | 0.874 |
| 3 | 0.431 | 0.522 | 0.998 |
| SP54+ | 0.423 | 0.511 | 0.971 |
| PREDNISONE | 0.464 | 0.559 | 1.039 |
| AVERAGE 3 | 0.439 | 0.531 | 1.003 |
| AVE 1 + AVE 2 | 0.400 | 0.482 | 0.958 |
| DIFFERENCE | 0.040 | 0.048 | 0.044 |

Ultraviolet spectra were also taken of the treated larch xylan composition designated as larch xylan sulfate II. Specifically, ultraviolet spectra of 0.33% larch xylan sulfate II - in aqueous pH 5.8, pH 12.2, and pH 1.5 solutions-are shown in Fig. 5.

$SP_{54}$m ---,---; of course, is known to be xylan sulphate, The ultraviolet spectra for $SP_{54}$ and the indicated larch xylan composition being very similar, with both having maxima at 262 nm. and minima at 245 nm., confirms that this treated larch xylan composition is indeed also a xylan sulfate.

The infrared spectra for $SP_{54}$ xylan sulfate, larch xylan sulfate I, and larch xylan sulfate II, are shown in Figs. 6, 7 and 8 respectively. The infrared spectrum for larch xylan is shown in Fig. 9.

The close similarity between the spectra of Figs. 6-8 further indicates sulfation of the larch xylan. As yet additional confirmation, the xylan sulfate spectra of Figs. 6-8 include strong absorption bands, at 1240 and $800^{cm-1}$, which are characteristic of sulfate groups attached to polysaccharides. These bands are lacking in Fig. 9, for the larch xylan not treated with the previously discussed sulfation process.

3. Proton and Carbon Thirteen Nuclear Magnetic Resonance Analyses of $SP_{54}$ Xylan Sulfate and Larch Xylan Sulfate II

Proton nuclear magnetic resonance ($H^1NMR$) and carbon thirteen nuclear magnetic resonance ($C^{13}NMR$) analyses were conducted for each of $SP^{54}$ xylan sulfate and larch xylan sulfate II. For these analyses, solutions of the xylan sulfates in deuterium oxide ($D_2O$) were utilized.

In the $H^1NMR$ analyses for both xylan sulfates, 3-(trimethylsilyl)-1-propanesulfonic acid, resonating at 0 and 40 ppm, was utilized as an internal standard. The large peak at 4.66 ppm in the larch xylan sulfate II analysis, and at 4.7 in the $SP_{54}$ analysis, is the monodeutero water peak.

The data discussed below demonstrate that the nuclear and carbon thirteen magnetic resonance analyses for $SP_{54}$ xylan sulfate are consistent; further, both of these analyses confirm that xylan sulfate is characterized by the structure as noted herein. However, comparison of these analyses with proton and carbon thirteen nuclear magnetic resonance analyses for larch xylan sulfate II reveals differences between these xylan sulfates.

Fig. 10 depicts the proton nuclear magnetic resonance spectrum of $SP_{54}$ xylan sulfate. Characteristic chemical shifts were assignable to the peaks for this spectrum.

The values corresponding to the peaks obtained from the proton nuclear magnetic resonance analysis for $SP_{54}$ xylan sulfate are tabulated in Table 2.

TABLE 2

| PPM | INTENSITY |
|---|---|
| 5.47 | 17.636 |
| 4.83 | 4.229 |
| 4.79 | 14.571 |
| 4.76 | 7.011 |
| 4.76 | 6.142 |
| 4.73 | 4.395 |
| 4.70 | 656.082 |
| 4.65 | 5.182 |
| 4.64 | 4.848 |
| 4.57 | 4.000 |
| 4.56 | 4.031 |
| 4.47 | 18.153 |
| 4.41 | 9.862 |
| 4.37 | 4.153 |
| 3.97 | 15.325 |
| 3.93 | 5.274 |
| 3.92 | 4.191 |
| 3.91 | 4.785 |
| 3.87 | 11.245 |
| 3.83 | 9.399 |
| 3.49 | 4.188 |
| 3.47 | 6.815 |

In the $H^1NMR$ spectrum, the large peak at 4.7 ppm is the monodeutero water peak. Due to incomplete sulfation and the presence of uronide side chains on the xylan, multiple peaks are present for each proton on the sugar rings. The sulfation of a ring hydroxyl causes an upfield shift for the adjacent proton on the same ring carbon as contains the hydroxyl sulfated. Integration of the proton NMR peaks accounts for the anomeric C-1 protons at 5.1-5.2 ppm, the C-5 methylene protons, the C-2 and C-3 ring hydrogens (where sulfucation occurs) and the proton of the glucosidically linked C-4 position.

Fig. 11 shows the carbon thirteen nuclear magnetic resonance spectrum of $SP_{54}$ xylan sulfate. As with the proton nuclear magnetic resonance, the carbon 13 spectra are clear and especially assigned.

Values corresponding to the peaks obtained from the carbon 13 nuclear magnetic resonance analysis of $SP_{54}$ xylan sulfate are tabulated in Table 3.

TABLE 3

| PPM | INTENSITY |
| --- | --- |
| 102.02 | 14.270 |
| 77.09 | 5.980 |
| 76.95 | 16.327 |
| 75.51 | 18.031 |
| 75.03 | 16.537 |
| 74.05 | 5.356 |
| 73.83 | 4.179 |
| 61.59 | 12.428 |
| 61.08 | 4.185 |

The ($^{13}C$)NMPR spectrum depicts differentiated ratios of sulfated and unsulfated secondary carbons bearing alcoholic functions. The anomeric C-1 at 102 pp, ring methylene carbon at C-5, the C-2 and C-3 secondary alcohol carbons, and the C-4 with its glycosidic linkage to the next xylose unit are assigned with reasonable probability. Smaller carbon signals probably belonging to the uronic acid side chains are present, but with sulfate substitutions and low concentrations, are difficult to assign.

The portion and carbon thirteen nuclear magnetic resonance spectra for larch xylan sulfate II are depicted in Fig. 12 and Fig. 13, respectively. The values corresponding to the peaks for these spectra are similarly set forth below in Table 4 and Table 5.

TABLE 4

| PPM | INTENSITY |
|---|---|
| 5.24 | 3.90 |
| 5.19 | 3.86 |
| 5.15 | 4.31 |
| 5.07 | 4.82 |
| 5.05 | 3.46 |
| 4.94 | 9.41 |
| 4.90 | 10.66 |
| 4.77 | 9.29 |
| 4.72 | 14.57 |
| 4.71 | 14.38 |
| 4.71 | 14.41 |
| 4.66 | 1083.02 |
| 4.61 | 10.47 |
| 4.55 | 9.91 |
| 4.53 | 10.51 |
| 4.45 | 11.92 |
| 4.37 | 15.66 |
| 4.14 | 10.03 |
| 4.03 | 5.36 |
| 3.93 | 14.59 |
| 3.82 | 18.00 |
| 3.67 | 7.05 |
| 3.65 | 10.28 |
| 3.63 | 9.95 |
| 3.61 | 6.03 |
| 3.58 | 3.48 |
| 3.48 | 4.16 |
| 3.46 | 2.87 |
| 3.34 | 7.27 |
| 1.19 | 6.85 |
| 1.17 | 14.75 |
| 1.15 | 9.04 |

TABLE 5

| PPM | INTENSITY |
|---|---|
| 85.21 | 3.05 |
| 82.98 | 7.08 |
| 82.24 | 6.25 |
| 62.69 | 6.31 |
| 61.49 | 6.34 |
| 61.09 | 5.99 |
| 60.37 | 6.63 |
| 59.90 | 7.70 |
| 58.94 | 7.77 |
| 58.54 | 7.22 |
| 57.36 | 6.97 |
| 56.93 | 8.01 |
| 55.93 | 20.28 |
| 55.63 | 20.76 |
| 54.47 | 5.54 |
| 53.51 | 6.06 |
| 53.07 | 3.50 |
| 52.06 | 4.06 |
| 49.40 | 10.46 |
| 45.62 | 2.06 |
| 45.22 | 2.22 |
| 42.10 | 2.59 |
| 41.61 | 2.04 |

For the larch xylan sulfate II analyses, the principal peaks are listed for each spectrum. Due to the presence of xylose, arabinose, mannose, glucose, and glucuronic acid, a very complex pattern results; the complexity of the xylan sulfate renders individual peak assignments difficult.

Particularly as to the anomeric carbon signals, for the $H^1$NMR, these are in the range of 5.0-5.5 ppm. In the $C^{13}$NMR these signals appear in the 75-85 ppm range, and are shifted downfield, possibly because of shielding from the sulfate groups.

Regarding the comparison of $H^1$ and $C^{13}$ NMR analyses for larch xylan sulfate II and $SP_{54}$ xylan sulfate, the $H^1$NMR analyses provide similar readings. However, for $C^{13}$NMR, the results differ considerably.

In the $C^{13}$NMR spectra for larch xylan sulfate II and $SP_{54}$ xylan sulfate, the greatest difference is in the location of the anomeric carbon signals. For $SP_{54}$, these signals are at 102 ppm, and for larch xylan sulfate, at 82-85 ppm. The spectrum from 90-190 ppm was scanned, but no signals were detected; the reason may be the complexity of the larch polymer.

2. High Pressure Liquid Chromatography of the $SP_{54}$ and Larch Xylan Sulfates

Samples of $SP_{54}$, in both powder and vial form, of larch xylan sulfate I, and of larch xylan sulfate II, were all subjected to high pressure liquid chromatography (HPLC). The elution pattern of all four were compared to three standards: glucose polymer, having a molecular weight of 800; dextran, having a molecular weight of 10,000; and blue dextran, having a molecular weight of 2,000,000.

10% aqueous solutions of the samples were filtered through a 0.2 $\mu$ filter. Columns for the gel permeation HPLC were two Spherogel TSK 2000 PW, 7.5 x 30 cm., followed by a Zorbax Bioseries, GF-250, 10.0 x 25 cm., with deaerated water used as the mobile phase; the eluent was monitored with a refractive index detector, and an ultraviolet detector at 254 mm.

Figs. 14-17 show the HPLC results, respectively, for $SP_{54}$ vial (i.e., stock solution), $SP_{54}$ powder, larch xylan sulfate I, and larch xylan sulfate II; Table 6 sets forth the data from the refractive index detector. The distance from the injection of the sample is reported in mm.; in gel permeation chromatography, the larger samples are eluted first.

TABLE 6

| Sample | Beg | Peak Max | End (mm) | Height |
|---|---|---|---|---|
| Glucose polymer, MW 800 | 64 | 81 | 107 | 155 |
| Dextran, MW 10,000 | 54 | 66 | 99 | 49 |
| $SP_{54}$, vial | 47 | 61 | 88 | 41 |
| $SP_{54}$, powder | 49 | 59 | 100 | 27 |
| Larch xylan sulfate, I | 47 | 60 | 97 | 93 |
| Larch, xylan sulfate, II | 47 | 58 | 94 | 79 |
| Blue dextran, MW 2,000,000 | 45 | 50 | 99 | 49* |

*This sample was more dilute and was detected in the ultraviolet.

The xylan sulfates all eluted between the dextran and the blue dextran. While their peak maxima are quite similar, the peak heights of the $SP_{54}$ samples are lower than those of larch xylan sulfate I and larch xylan sulfate II, thereby allowing an interpretation that $SP_{54}$ xylan sulfate is characterized by a broader molecular weight distribution. The peak for larch xylan sulfate II trailed more that of larch xylan sulfate I, indicating the presence of more lower molecular weight material in the former.

3. Comparison of Sugar Ratios for Larch Xylan Sulfate II and $SP_{54}$ Xylan Sulfate

Larch xylan sulfate II was subjected to a series of reactions to determine sugar ratios for this polysaccharide.

Specifically, an amount of larch xylan sulfate II was hydrolyzed in 2M trifluoroacetic acid. The alditol acetates of the hydrolyzate were prepared, and the derivatized hydrolyzate was run on the gas chromatograph.

The resulting elution pattern was compared to the retention times of the alditol acetates of the individual sugars, and the relative quantity of the sugars was calculated from the response factors of the individual sugars. The unknown peaks were based upon the average response factors of all the sugars combined.

The glucuronic acid in the larch xylan sulfate II was determined calorimetrically, using m-phenylphenol as the color reagent, while the total carbohydrate was determined by the phenol/sulfonic acid method. The remainder of the larch xylan sulfate II are the sodium sulfate ester and water of hydration.

The percentages of the individual sugars were calculated by combining the ratios from the alditol acetate derivatives with the data from the carbohydrate and glucuronic acid determinations. These numbers are considered to have meaning as ratios only, because of errors inherent in the hydrolysis and derivatization, and errors resulting from combining results from different methods.

The ratios obtained from the indicated reactions and calculations are set forth in Table 7.

TABLE 7

| | | |
|---|---|---|
| RESULTS: | | |
| Total carbohydrate | | 48.6% |
| Individual sugars | | |
| | arabinose | 3.6 |
| | xylose | 10.5 |
| | unknown | 3.5 |
| | mannose | 18.8 |
| | glucose | 7.1 |
| | unknown | 3.1 |
| | glucuronic acid | 2.0 |
| | Total | 48.6 |

To confirm the relative proportions of sugars indicated to be present in the larch xylan sulfate II, another amount of this polysaccharide composition was subjected to methanolysis. The methyl glycosides were derivitized as the trimethylsilyl (TMS) ethers.

TMS derivatives of the methyl glycosides of xylose, mannose, galactose, glucuronic acid, and B-pheylglucose were prepared as standards, and run on the gas chromatograph to determine the relative retention times and response factors. The B-phenylglucose was included as an internal standard in the preparation of the TMS derivative of the methanolysis mixture.

The recovery in the derivatization was 23%. The relative ratios of the sugars from this methanolysis procedure are set forth below, in Table 8; however, because of the low yield of methanolysis derivative in depolymerizing the sulfated larch xylan, these ratios should be considered less quantitatively reliable than those from the hydrolysis and alditol acetate derivatives above. They are qualitatively indicative of the larch xylan sulfate's sugar composition.

TABLE 8

| | |
|---|---|
| methyl xyloside | 3.0 |
| methyl mannoside | 10.0 |
| methyl glucuronoside methyl ester | 2.2 |
| methyl glucoside | 1.8 |

The sugar ratios for $SP_{54}$ xylan sulfate are reported in the previously cited BAYOL et al., U.S. Patent No: 4,713,373. These values are reproduced below in Table 9.

TABLE 9

| | |
|---|---|
| xylose | 30-40% |
| glucuronic acid | 4-6 |

The difference in sugar content between larch xylan sulfate II and $SP_{54}$ xylan sulfate is significant. The larch polysaccharide composition contains both pentoses and hexoses, and has only 10.5% xylose, compared to 30-40% reported for $SP_{54}$. Further, $SP_{54}$ is characterized by a ratio of 1 uronic acid group for every 8 to 10 xyloses. In larch xylan sulfate II, this ratio is about 1 to 5; however, the ratio of uronic acid groups to total sugars is about 1 to 20.

4. Elemental Analyses of $SP_{54}$ Xylan Sulfate and Larch Xylan Sulfate II

Distinction between the xylan sulfates is further evidenced by elemental analyses for $SP_{54}$ xylan sulfate and larch xylan II. These elemental analyses were calculated as set forth below.

Specifically, these elemental analyses are provided as percentages, and are provided as averages of duplicate analyses. Molar ratios were calculated for a pentose, a hexose, and a combination of pentose and hexose, C = 5.64, which corresponds to the sugar distribution determined by derivation and gas chromatography.

The analyses reveal approximately 1.5 sulfates per sugar moiety, confirming the presence of both monosulfated and disulfated sugars. The analyses further indicated the presence of approximately on water of hydration per sugar moiety.

The elemental analyses results are set forth for xylan sulfate larch II in Table 10; for $SP_{54}$ xylan sulfate, in Table 11. The differences between these xylan sulfate compositions can be discerned from comparison of the respective data.

TABLE 10

| Atom or Group | Analytical Results % | Calculated Molar Hexose | Pentose | Ratios Pentose +Hexose | Formula Ratios |
|---|---|---|---|---|---|
| C | 19.08 | 6.00 | 5.00 | 5.64 | 5 or 6 |
| H | 3.06 | 11.51 | 9.59 | 10.81 | 9 to 11 |
| S | 14.22 | 1.66 | 1.39 | 1.56 | 1 to 2 |
| $SO_4$ | 35.03 | 1.38 | 1.15 | 1.30 | 1 to 2 |
| Na | 11.91 | 1.98 | 1.65 | 1.86 | 1 to 4 |
| $H_2O$ | 6.20 | 1.30 | 1.08 | 1.22 | 1 to 2 |

TABLE 11

| Atom or Group | Analytical Results % | Calculated Molar Ratios Pentose | Formula Ratios |
|---|---|---|---|
| C | 16.30 | 5.00 | 5 |
| | 16.49 | 5.06 | |
| H | 2.60 | 9.49 | 6 to 8 |
| | 2.58 | 9.42 | |
| S | 16.86 | 1.94 | 1 to 2 |
| | 16.69 | 1.92 | |
| $SO_4$ | 39.14 | 1.50 | 1 to 2 |
| ASH | 38.74 | 1.48 | |

The elemental analyses are reported in per cent and is the average of duplicate analyses. Molar ratios were calculated and compared to theoretical ratios. The molar ratios are calculated for a pentose, a hexose, and a combination of pentose and hexose, C = 5.64, that corresponds to the sugar distribution determined by derivatization and gas chromatography. There are approximately 1.5 sulfates/sugar moiety, indicating the presence of mono-and disulfated sugars in the polymer. There is approximately one water of hydration per sugar moiety.

COMPARATIVE ACTIVITY OF POLYSACCHARIDE AND POLYSACCHARIDE PLUS STEROID COMPOSITIONS

The following procedures demonstrate the effect of certain compositions of the invention, and of heparin compositions, on one particular physiological mechanism, i.e., angiogenesis, induced by different growth factors.

1. Fractionation of $SP_{54}$ Xylan Sulfate

The xylan sulfate fractionation sample was 1.5 g. of $SP_{54}$ powder, with water as the solvent. Five specific weight average molecular weight fractions were obtained by gel permeation chromatography.

For the purpose of determining the feasibility of $SP_{54}$ fractionation, a small amount (i.e., 0.5 g.) of $SP_{54}$ was analyzed by gel-filtration, using the resin bio Gel P-6 on a 1.5 cm. diam. X 50 cm. column. This resin has a fractionation range of 1000-6000 daltons.

Void and inclusion volumes were determined by chromatographing bovine serum albumin (void) and mannose (inclusion) over the same column. The sample was eluted with water. Fractions were collected and the carbohydrate content of each fraction was determined by phenol/sulfuric acid color reaction.

The resulting elution profile, shown in Fig. 18, clearly demonstrates the heterogeneity of the sample. Because a large portion of the sample elutes later than mannose, the sample may have interacted with the resin; however, these results demonstrated that separation of the sample into at least five fractions is possible.

1.0 g. of $SP_{54}$ was next applied to a 28 X 90 cm. Bio Gel P-6 column. As before, the sample was eluted with water, and assayed for carbohydrate, being pooled as shown in Fig. 19.

Again the heterogeneity was evident. The sample was separated into five fractions, I-V, as indicated in Fig. 19, and further analyses were performed on each fraction.

Specifically, for the purpose of determining whether the fractions were separated according to molecular weight, they were re-chromatographed on the smaller Bio Gel P-6 column; aliquots of end fraction were applied to the column. To prevent interaction with the resin, chromatography was performed with 0.5M NaCl; elution was with 0.5M NaCl, and void volume (t) was determined with BSA.

The elution profiles of the five fractions are shown in Fig. 17. The molecular weights of these fractions were calculated as hereinafter discussed; the weight average molecular weights thus calculated are set forth in Fig. 20, each corresponding to its respective fraction.

These results indicate that Fractions I-V were indeed separated according to molecular weight. The appearance of the second peak in Fraction I indicates that, in addition to containing material similar to that of Fraction II, Fraction I also contains other high molecular weight material.

The fractions were read at 490 nanometers, with these readings consisting of the large peak shown in Fig. 21. An ultraviolet scan of the $SP_{54}$ solution showed a maximum at 232 nanometers; the fractions were also read directly in the ultraviolet region at 232 nanometers.

The small peak in Fig. 21 corresponds to these readings. This peak is probably due to ultraviolet resonance of the sulfate group.

The carbohydrate peak extends beyond the sulfate peak. This could be due either to a lower degree of sulfation of these fractions, or to less sensitivity of the absorbance maximum at 232 nanometers; that is, the molar absorbance at 232 nanometers could be less than the molar absorbance of the carbohydrate complex at 490 nanometers. The different possibilities could be determined from the sulfate content of the fractions.

The fractions were divided as indicated in Fig. 21. The yield of the fractions is as set forth in Table 12.

TABLE 12

| | Mass | Percent | |
|---|---|---|---|
| I | 37 mg. | 2.4 | |
| II | 68 mg. | 4.5 | |
| III | 126 mg. | 8.4 | Total Recovery 66.8% |
| IV | 65 mg. | 4.3 | |
| V | 708 mg. | 47.2 | |

Elemental analysis of the xylan sulfate fractions yielded the results set forth in Table 13.

TABLE 13

| Fraction | Carbohydrate(mg) | Sulfate(mg) | Total(mg) | %Sulfate |
|---|---|---|---|---|
| I | 2.25 | 5.0 | 7.3 | 68.9 |
| II | 3.90 | 7.4 | 11.3 | 65.5 |
| III | 4.65 | 8.0 | 12.7 | 63.2 |
| IV | 4.75 | 11.0 | 15.8 | 69.8 |
| V | 6.00 | 14.0 | 20.0 | 70.0 |

The molecular weight of these fractions was determined by separation on a Bio Gel P-6 column, in the manner as previously discussed. The column was calibrated for the purpose of effecting this determination.

Polystyrene sulfonate molecular weight standards having the range 1,580-47,200 were used to calibrate the gel filtration columns in the TSK-PWXL series: 2,500; 3,000; 4,000. The solvent used was 10% acetonitrile in 0.2M NaCl. Ten runs were used to develop the standard curve. The equation with the closest fit to these

points had a correlation coefficient or R-squared value of 0.98.

The closer this number is to 1, the greater the reliability of the curve; accordingly, this value represents a close fit. The molecular weights of the fractions were calculated from the equation, and represent the weight average molecular weights.

The molecular weights of these fractions were thus determined as set forth in Table 14.

TABLE 14

| Fraction | Molecular Weight (Weight Average) |
|---|---|
| I | 8820 |
| II | 5290 |
| III | 4370 |
| IV | 3900 |
| V | 4210 |

These fractions were tested for endotoxin using the Limulus Amebocyte Lysate assay. The results are set forth in Table 4.

TABLE 15

| Fraction | Endotoxin units/ug |
|---|---|
| I | 14.3 |
| II | 2.9 |
| III | 2.9 |
| IV | 14.3 |
| V | 2.9 |

2. Measuring Effect of Compositions and Combinations Thereof on Specific Growth Factors

In view and in vitro assays were conducted, measuring effects of different polysaccharides, steroids, polysaccharide combinations, and combinations of steroids with polysaccharide fragments and with one or more saccharide, on growth factors. The substances used in these assays are listed below, with their acronyms or abbreviations set forth in these Tables.

Growth Factors (GF's)

Endothelial Cell Growth Factor (ECGS)
Acidic Fibroblast Growth Factor (aFGF)
Basic Fibroblast Growth Factor (bFGF)
Platelet Derived Growth Factor (PDGF)
Transforming Growth Factor - Beta (TGF-Beta)
Epidermal Growth Factor (EGF)
Tumor Necrosis Factor - Alpha (TNF-Alpha)

Polysaccharides

The xylan sulfate, and fractions thereof, are those as previously discussed with reference to the experimental procedure concerning fractionation of xylan sulfate.

Heparin (sodium salt)-Hep
$SP_{54}$ Xylan Sulfate - $SP_{54}$
Xylan Sulfate Fraction I - weight average molecular weight 8820($SP_{54}$(8820))
Xylan Sulfate Fraction II - weight average molecular weight 5290($SP_{54}$(5290))
Xylan Sulfate Fraction III - weight average molecular weight 4370($SP_{54}$(4370))
Xylan Sulfate Fraction IV - weight average molecular weight 3900($SP_{54}$(3900))
Xylan Sulfate Fraction V - weight average molecular weight 4210($SP_{54}$(4210))

Larch Xylan Sulfate II- LXS II

Steroids (St's)

Hydrocortisone (HC)
Cortexolone (C)

Carrier/Release Vehicle

Agarose (Ag)

a. In Vitro Assay Comparing Effects of Compositions on Inducement of Angiogenesis by Growth Factors

Various compositions were tested in vitro for their affect on angiogenesis induced by different growth factors. The test subjects were golden syrian hamsters (Mesocricetus auratus). All were young adults, having an initial weight of 100 grams ± 50 grams.

Various substances were administered, as set forth in Tables A-D hereinafter, in the following combinations, in the following amounts:

when growth factor was administered alone - 10 ug GF, dissolved in Ag;

when growth factor was administered with heparin - 10 ug GF and 2 units Hep, dissolved in Ag;

when growth factor was administered with heparin and a steroid - 10 ug GF, 2 units Hep, 100 ug steroid, absorbed in Ag;

when growth factor was administered with $SP_{54}$ or $SP_{54}$ (fraction) - 10 ug GF, 12 ug $SP_{54}$ or $SP_{54}$ (fraction) in Ag; and

when growth factor was administered with $SP_{54}$ or $SP_{54}$ (fraction), and a steroid - 10 ug GF, 12 ug $SP_{54}$ or $SP_{54}$ (fraction), and 100 ug St.

Administration was by injection, with a needle and syringe, at a single submucocutaneous site in the left cheek pouch of each hamster. Each of these combinations of substances was administered to a group of four hamsters, two males and two females; during administration, and during periodic examinations of the test sites, the hamsters were given an intraperitoneal anaesthetic mixture comprising 50.0 mg./kg. ketamine HCl, 5.0 mg./kg. xylazine, and 1.0 mg./kg. acepromazine.

Several different types of observations were taken in determining the degree of angiogenesis resulting from the injections.

On the fifth day after injection, the hamsters were examined under anesthesia, through an operating microscope; photographs were also taken through the microscope, and studied. The animals were euthanized, with the test sites removed, fixed in neutral buffered formalin, pinned on wax, sectioned at 5 u, and stained, after which they were examined with light microscopy. These pinned test sites were also photographed, with these photographs being studied.

From these analyses, the degree of angiogenesis observed in each hamster was determined according to the following system of measurement;

0 = no new vessels;

1 = minimal ramification of vessels in vicinity of injection site;

2 = new vessels reach injection site, area involved is less than 50% of periphery of injection site;

3 = many new tortuous vessels that reach and cross injection site in greater than 50% of periphery of injection site; and

4 = many new tortuous vessels, hemorrhages, invasion of vessels in areas remote from injection site.

The higher the number, the greater the degree of angiogenesis. This system of measurement, designated the Hamster Cheek Pouch Assay, is described in SCHREIBER et al., Science, Vol 232, June 6, 1986, p. 1250.

In Tables A - D hereinafter, each measurement represents the score of a four hamster group, calculated by adding the scores of the four hamsters in the group. Thus, for instance, the highest possible score is 16, denoting a maximum angiogenesis score of 4 for each of the four hamsters in the group.

The first number in each of Tables A - D lists the total score obtained from administering the designated growth factor alone; the subsequent numbers in the row give the total score for that growth factor in concert with the specified substances. Comparing each of these subsequent scores with the first score in the row will indicate the affect, of the substances corresponding to those scores, on the particular growth factor function being measured, i.e., in this case, angiogenesis.

For instance, if the total score obtained from the use of such substances in concert with the growth factor is lower than that obtained from the growth factor alone, these substances have inhibited the activity, i.e.,

angiogenesis, of the growth factor in question. If the score is instead greater, the growth factor activity has been stimulated. Of course, if there is no change in the score, growth factor activity has not been affected.

TABLE A

| | GF | GF+Hep | GF+$SP_{54}$ | GF+Hep+HC | GF+$SP_{54}$+HC |
|---|---|---|---|---|---|
| ECGS | 16 | 12 | 12 | 11 | 8 |
| aFGF | 5 | 8 | 10 | 7 | 6 |
| bFGF | 6 | 11 | 6 | 7 | 9 |
| PDGF | 8 | 8 | 7 | 9 | 10 |
| TGF-Beta | 8 | 10 | 9 | 7 | 12 |
| EGF | 7 | 9 | 3 | 8 | 9 |
| TNF-Alpha | 9 | 10 | 9 | 13 | 11 |

TABLE B

| | GF | GF + $SP_{54}$ | GF + $SP_{54}$ (8820) | GF + $SP_{54}$ (5290) | GF + $SP_{54}$ (4370) | GF + $SP_{54}$ (3900) | GF + $SP_{54}$ (4210) |
|---|---|---|---|---|---|---|---|
| ECGS | 16 | 12 | 8 | 9 | 9 | 7 | 11 |
| aFGF | 5 | 10 | 9 | 7 | 11 | 7 | 7 |
| bFGF | 6 | 6 | 10 | 12 | 12 | 7 | 10 |
| FDG | 8 | 7 | 12 | 10 | 11 | 10 | 11 |
| TGF-Beta | 8 | 9 | 14 | 12 | 9 | 12 | 10 |
| EGF | 7 | 3 | 6 | 12 | 11 | 4 | 11 |
| TNF-Alpha | 9 | 9 | 8 | 9 | 11 | 5 | 7 |

TABLE C

| | GF | GF + Hep + HC | GF + $SP_{54}$ + HC | GF + $SP_{54}$ (8820) + HC | GF + $SP_{54}$ (5290) + HC | GF + $SP_{54}$ (4370) + HC | GF + $SP_{54}$ (3900) + HC | GF + $SP_{54}$ (4210) + HC |
|---|---|---|---|---|---|---|---|---|
| ECGS | 16 | 11 | 8 | 6 | 7 | 7 | 7 | 9 |
| aFGF | 5 | 7 | 6 | 6 | 8 | 9 | 6 | 6 |
| bFGF | 6 | 7 | 9 | 9 | 5 | 8 | 14 | 9 |
| PDGF | 8 | 9 | 10 | 13 | 9 | 11 | 15 | 12 |
| TGF-Beta | 8 | 7 | 12 | 13 | 11 | 9 | 7 | 11 |
| EGF | 7 | 8 | 9 | 8 | 10 | 5 | 7 | 9 |
| TNF-Alpha | 9 | 13 | 11 | 10 | 12 | 8 | 10 | 8 |

TABLE D

| | GF | GF + Hep + NC | GF + Hep + C | GF + $SP_{54}$ + NC | GF + $SP_{54}$ + C | GF + $SP_{54}$ (8820) + NC | GF + $SP_{54}$ (8820) + C | GF + $SP_{54}$ (5290) + NC | GF + $SP_{54}$ (5290) + C | GF + $SP_{54}$ (4370) + NC | GF + $SP_{54}$ (4370) + C | GF + $SP_{54}$ (3900) + NC | GF + $SP_{54}$ (3900) + C | GF + $SP_{54}$ (4210) + NC | GF + $SP_{54}$ (4210) + C |
|---|---|---|---|---|---|---|---|---|---|---|---|---|---|---|---|
| ECGS | 16 | 11 | 12 | 8 | 6 | 6 | 10 | 7 | 8 | 7 | 9 | 7 | 6 | 9 | 10 |
| aFGF | 5 | 7 | 8 | 6 | 12 | 6 | 9 | 8 | 7 | 9 | 10 | 6 | 7 | 6 | 9 |
| bFGF | 6 | 7 | 9 | 9 | 11 | 9 | 11 | 5 | 9 | 8 | 9 | 14 | 7 | 9 | 10 |
| FDGF | 8 | 9 | 11 | 10 | 11 | 13 | 10 | 9 | 8 | 11 | 7 | 15 | 10 | 12 | 9 |
| TGF-Beta | 8 | 7 | 8 | 12 | 13 | 13 | 11 | 11 | 9 | 9 | 12 | 7 | 9 | 11 | 11 |
| EGF | 7 | 8 | 12 | 9 | 10 | 8 | 9 | 10 | 10 | 5 | 8 | 7 | 9 | 9 | 11 |
| TNF-Alpha | 9 | 13 | 8 | 11 | 11 | 10 | 13 | 12 | 12 | 8 | 9 | 10 | 11 | 8 | 10 |

One interesting observation to be taken from the data set forth in Tables A-D is the unexpected results obtained with certain combinations of the substances used. For instance, as previously discussed, the prior art discloses that the combination of heparin and steroid inhibits angiogenesis, as does xylan sulfate + steroid, but to a greater degree.

With reference to Table A, these substances provide just such a result in concert with ECGS. Used alone, ECGS effects the maximum angiogenesis possible according to the measuring system used, providing a total score of 16; heparin + hydrocortisone lessens the angiogenic effect of the growth factor, providing a total score of 11, and $SP_{54}$ + hydrocortisone lowers the total score still further, to 8.

However, with other growth factors, the results are directly opposed to what would be expected from the prior art. Again referring to Table A, the total score for bFGF is 6; $SP_{54}$ + hydrocortisone raises this measurement to 9, thereby stimulating angiogenesis, in direct contrast to the inhibition which would be expected from the prior art. Stimulation by $SP_{54}$ + hydrocortisone similarly results with the growth factors aFGF, PDGF, TGF-Beta, EGF, and TNF-Alpha.

Also worthy of note is the difference in results between certain growth factors. Again with reference to Table A, $SP_{54}$, as is known in the prior art, stimulates the angiogenic effect of aFGF; however, this same polysaccharide inhibits the angiogenic effect of EGF. Similarly, as shown in Table B, $SP_{54}$(3900) significantly inhibits ECGS and EGF, while stimulating, inter alia, TGF-Beta.

This phenomenon is also evident where steroid is included. As seen in Table C, the combination utilizing $SP_{54}$(8820) very strongly inhibits ECGS, but stimulates TGF- Beta; where the polysaccharide is $SP_{54}$(4370), aFGF is stimulated, but EGF is inhibited. In Table D, the combination of $SP_{54}$ + cortexolone severely inhibits ECGS, but stimulates several of the growth factors, particularly aFGF.

Similarly significant is the marked difference in activity which can be found between different $SP_{54}$ fractions. As shown in Table B, while $SP_{54}$ has no effect on bFGF, and $SP_{54}$(3900) provides only slight stimulation, $SP_{54}$ (8820), $SP_{54}$(5290), $SP_{54}$(4370), and $SP_{54}$(4210) all significantly stimulate the activity of this growth factor; EGF is significantly stimulated by $SP_{54}$(5290), but inhibited by $SP_{54}$ (3900). As to the combinations including steroid, Table C shows that the combination including $SP_{54}$ (5290) stimulates the angiogenic activity of EGF, while the combination with $SP_{54}$(4370) is an inhibitor of the growth factor; as seen in Table D, $SP_{54}$(8820) + cortexolone strongly stimulates TNF-Alpha, while $SP_{54}$(4370) + cortexolone has no effect thereon.

Differences can further arise from a change in steroid. Numerous examples can be found in Table D; for instance, with regard to bFGF and PDGF, the polysaccharide $SP_{54}$(3900) provides much greater inhibitory effect with hydrocortisone than with cortexolone; in contrast, cortexolone provides greater inhibition than hydrocortisone, in combination with this same polysaccharide, for the growth factors TGF-Beta and EGF.

The various comparisons discussed above are only representative examples in support of the points for which they are cited. Other such examples, also in support of these points, can be found in Table A-D.

In any event, these data demonstrate that the activity of the compositions of the invention is not predictable. These data further evidence that such activity is dependent upon a plurality of factors, including the identity of the various components of these compositions.

b. In Vitro Assay Comparing Effects of Compositions on Endothelial Cell Growth

The effect of various compositions on endothelial cell growth was tested in vitro, as set forth hereinafter in In Vitro Example 1 and In Vitro Example 2. As also subsequently discussed, one effect of particular significance is the difference in the activity where the only distinction is the presence of $SP_{54}$ xylan sulfate instead of larch xylan sulfate II.

Compositions and concentration for In Vitro Examples 1 and 2 are set forth below. In both Examples, a microliter plate was utilized, with $^{3}$H-thymidine uptake by actively dividing cells.

## In Vitro Example 1

Cell Count 70,000 cells/mL 7,000 cells/well

N = 6 Wells/Test Group

Concentrations

| | | |
|---|---|---|
| ECGS | 40 μg/mL | 8.8 μg/well |
| Heparin | 2mg/mL | 20 μg/well |
| HC | 10mg/mL | 100 μg/well(A) |
| $SP_{54}$ | 2.4mg/mL | 240 μg/well(A) |
| LXS II | 2.4mg/mL | 240 μg/well(A) |

Groups

ECGS+Heparin+HC(A)
ECGS+Hep+HC(A)+SP54(A)
ECGS+Hep+HC(A)+LXS II(A)

ECGS+Heparin
ECGS+Hep+SP54(A)
ECGS+Hep+LXSII(A)

ECGS+HC(A)
ECGS+HC(A)+SP54(A)
ECGS+HC(A)+LXSII
ECGS alone

Media contains 1 μg/mL HC or 0.22 μg/well

The results from In Vitro Example 1 are provided in Figure 22. In each instance where the only distinction is in the presence of $SP_{54}$ xylan sulfate instead of larch xylan sulfate II, there is a difference in activity. Specifically, in combination with heparin, $SP_{54}$ xylan sulfate inhibits endothelial cell growth 78%, while larch xylan sulfate II, in the same combination, inhibits such cell growth only 74%. Where the combination is with hydrocortisone, $SP_{54}$ xylan sulfate also provides greater inhibitory effect (i.e., 68%) than larch xylan sulfate II (60%). Where the common ingredients include both heparin and hydrocortisone, use of $SP_{54}$ xylan sulfate results in 91% inhibition; use of larch xylan sulfate II, 92%.

Another effect to be noted from this Example is that the presence of heparin alone provides virtually no effect on cell growth.

## In Vitro Example 2

Cell Count 150,000 cells/mL 15,000 cells/well

N = 6 Wells/Test Group

Concentrations

| | | |
|---|---|---|
| ECGS | 40 μg/mL | 8.8 μg/well |
| Hep | 2 mg/mL | 20 μg/well |
| HC | 1 mg/mL | 10 μg/well(B) |
| HC | 10 mg/mL | 100 μg/well(A) |
| SP54 | 0.24 mg/mL | 24 μg/well(B) |
| LXS II | 0.24 mg/mL | 24 μg/well(B) |

Groups

No ECGS (cells alone)
ECGS
ECGS+Hep
ECGS+HC(B)
ECGS+HC(A)

ECGS+Hep
ECGS+Hep+SP54(B)
ECGS+Hep+LXS II(B)

ECGS+HC(B)
ECGS+HC(B)+SP54(B)
ECGS+HC(B)+LXSII(B)
ECGS alone

**Media contains 1 μg/mL HC or 0.22 μg/well**

The results from In Vitro Example 2 are provided in Figure 23. In this Example also, there is a difference in activity between $SP_{54}$ and larch xylan sulfate II.

In contrast with In Vitro Example 1, at these polysaccharide concentrations, larch xylan sulfate II provides greater inhibition of cell growth than $SP_{54}$ xylan sulfate. In combination with heparin, larch xylan sulfate II provides 29% inhibition; $SP_{54}$ xylan sulfate, only 11%. In combination with hydrocortisone, larch xylan sulfate II provides 33% inhibition; $SP_{54}$ xylan sulfate, only 9%.

It would therefore appear that the effects of $SP_{54}$ xylan sulfate and larch xylan are dependent upon the concentrations of these polysaccharides. Yet additional effects can be ascertained from the results shown in In Vitro Example 2.

For instance, concentration, i.e., of hydrocortisone, is also demonstrated to be a factor with regard to the negative effect of this steroid on endothelial cell growth. As with In Vitro Example 1, heparin is found to have the same effect on cell growth as ECGS alone. Further, the data indicate that ECGS is required for proliferation in endothelial cell growth; the culture without ECGS, i.e., comprising cells alone, demonstrates 88% inhibition, as compared to the ECGS + Hep or ECGS cultures.

Although the invention has been described with respect to particular means, materials, and embodiments, it is understood that the invention is not limited to the particulars disclosed, and extends to all equivalents within the scope of the claims.

## Claims

1. A composition for modulating the effect or effects of an animal growth factor, especially a human growth factor, other than for the stimulation of aFGF, in vivo or in vitro, comprising a xylan, at least partially sulphated, and preferably a suitable carrier therefor.

2. A composition according to claim 1 for inhibiting the effect or effects of the growth factor.

3. A composition according to claim 1 or 2, wherein the growth factor is ECGS, aFGF, bFGF, PDGF, TGF-β, EGF and/or TNF-α.

4. A composition according to any preceding claim, wherein the xylan sulphate has an average molecular weight of around 8820, 5290, 4370, 3900 or 4210.

5. A composition according to any preceding claim, further comprising a steroid, provided that the composition is not for the inhibition of angiogenesis when comprising unfractionated xylan sulphate.

6. A composition according to claim 7, wherein the steroid is cortisone, hydrocortisone, 11 alpha epihydrocortisol, tetrahydro S, 17 alpha hydroxy progesterone, delta-cortisone or cortexolone.

7. A composition according to any preceding claim, for stimulating the effect or effects of the growth factor, especially when dependent on either of claims 5 or 6.

8. A composition as defined in any preceding claim, for inhibiting fibroblast proliferation, particularly when the composition composition comprises essentially no steroid, and especially wherein the xylan sulphate is derived from beechwood.

9. A composition as defined in any of claims 5 to 7, for stimulating angiogenesis.

10. A composition according to any preceding claim, further comprising the relevant growth factor or factors.

11. A composition according to any preceding claim, wherein the xylan sulphate is derived from beechwood or larch.

12. A composition according to any preceding claim, characterised in that it is adapted for pharmaceutical administration.

13. A composition according to claim 12, adapted for localised administration, especially to sites of undesirable fibroblast proliferation, or where angiogenesis is desired.

14. Use of a specific average weight fraction of xylan sulphate in therapy, especially as a composition as defined in any preceding claim.

15. Use of a composition as defined in any of claims 1 to 14, in the manufacture of a medicament for the treatment of a condition which condition: is treatable by the reduction of fibroblast proliferation; or is treatable by the stimulation of angiogenesis; or is treatable by the modulation of the effect or effects of a growth factor as defined in any preceding claim.

16. Use according to claim 15, wherein the condition is improper tissue production of, or improper physiological response to, the growth factor.

17. Use according to claim 15 or 16, wherein the condition is an excess of, or an excessive response to, the growth factor.

18. Use according to claim 15 or 16, wherein the condition is an insufficiency of, or an insufficient response to, the growth factor.

19. A kit comprising the essential elements of any preceding claim.

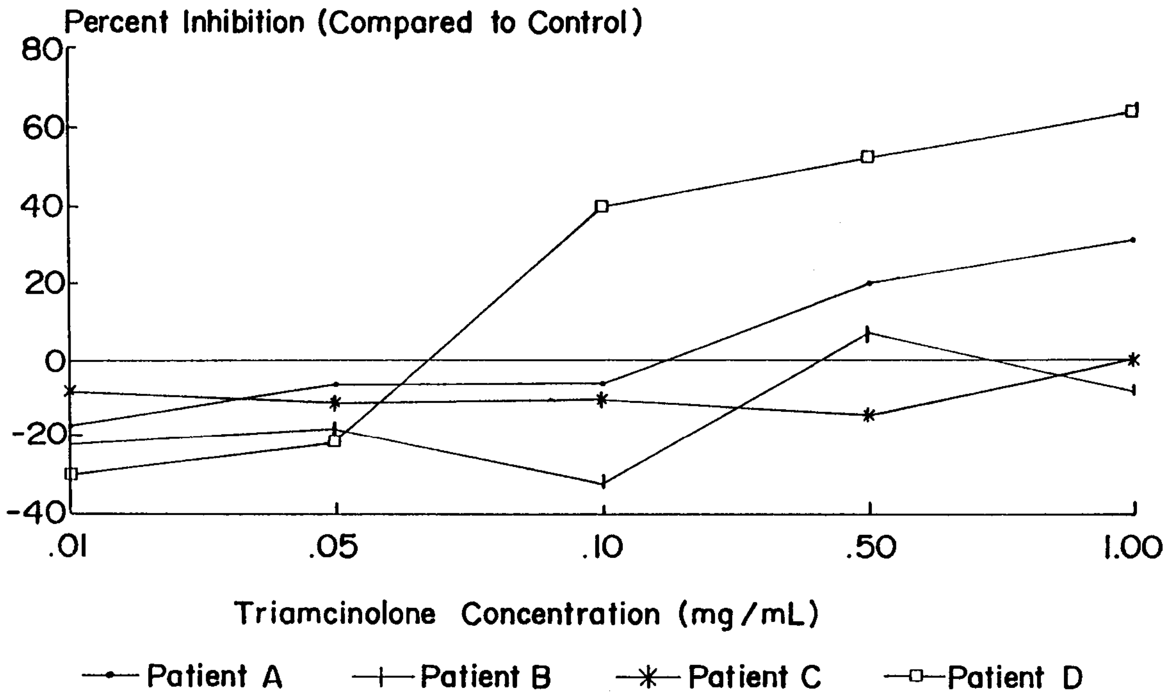
Percent Inhibition (Compared to Control)
80
60
40
20
0
-20
-40
.01
.05
.10
.50
1.00
Triamcinolone Concentration (mg/mL)
Patient A
Patient B
Patient C
Patient D

Fig. 1

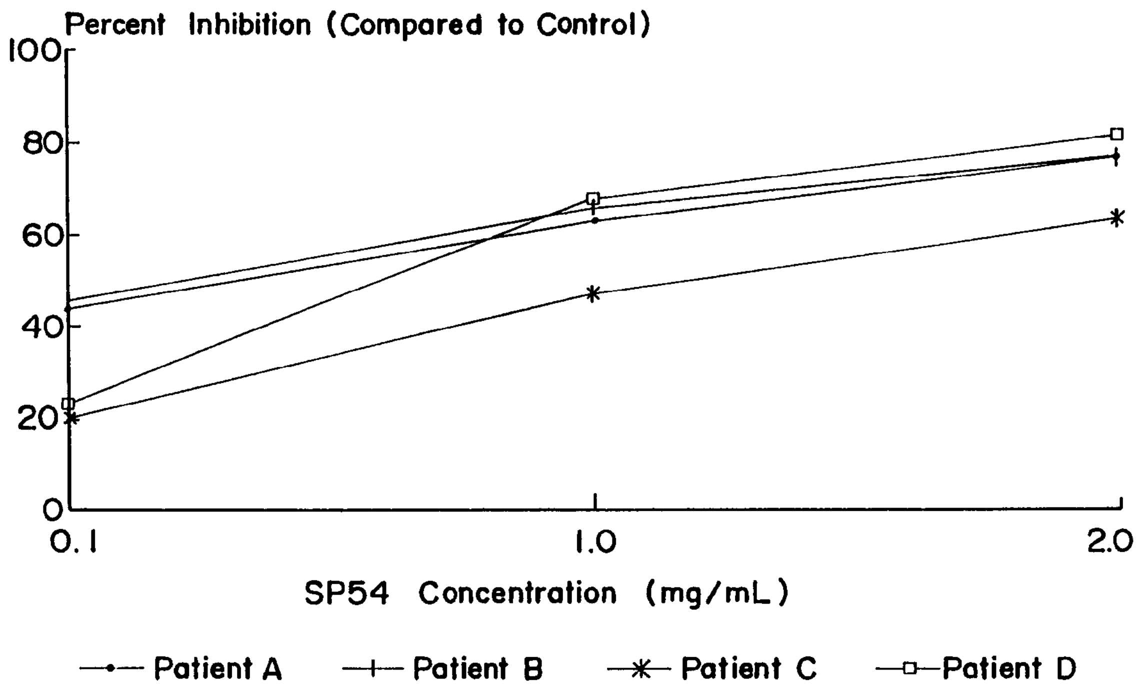
Percent Inhibition (Compared to Control)
100
80
60
40
20
0
0.1
1.0
2.0
SP54 Concentration (mg/mL)
Patient A
Patient B
Patient C
Patient D

Fig - 2

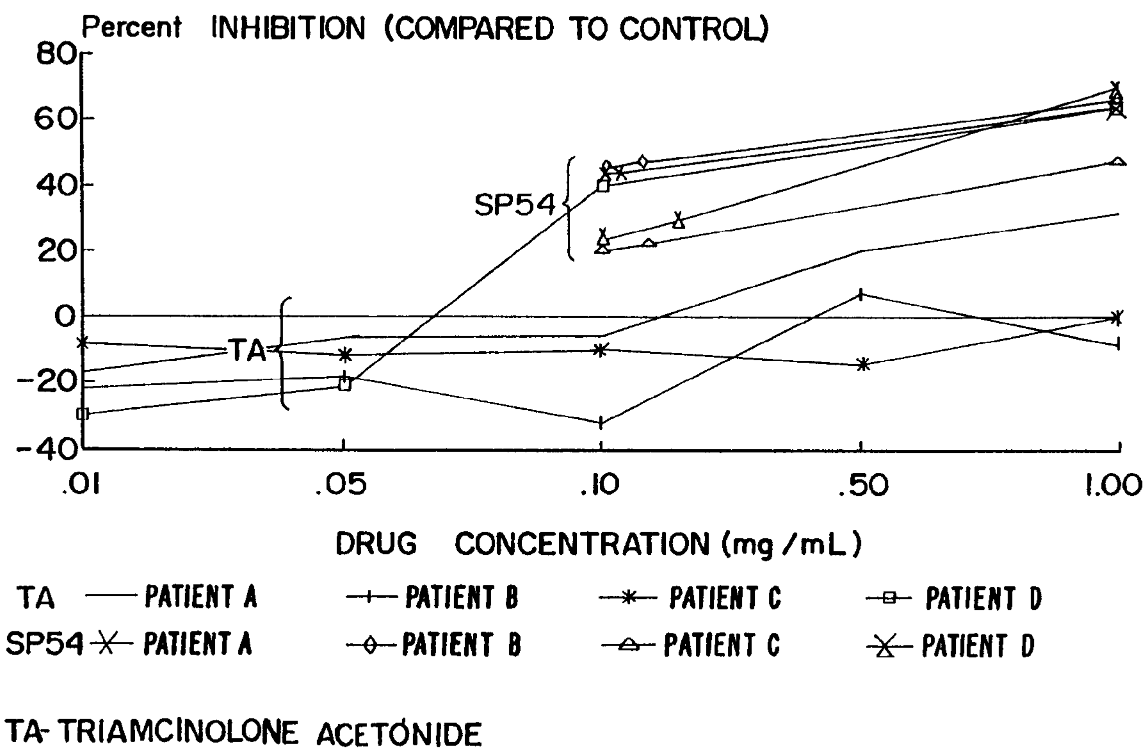
Percent INHIBITION (COMPARED TO CONTROL)
80
60
40
20
0
-20
-40
SP54
TA
.01
.05
.10
.50
1.00
DRUG CONCENTRATION (mg / mL)
TA — PATIENT A
—+— PATIENT B
—*— PATIENT C
—□— PATIENT D
SP54 —X— PATIENT A
—◇— PATIENT B
—△— PATIENT C
—X— PATIENT D
TA- TRIAMCINOLONE ACETÓNIDE

Fig. 3

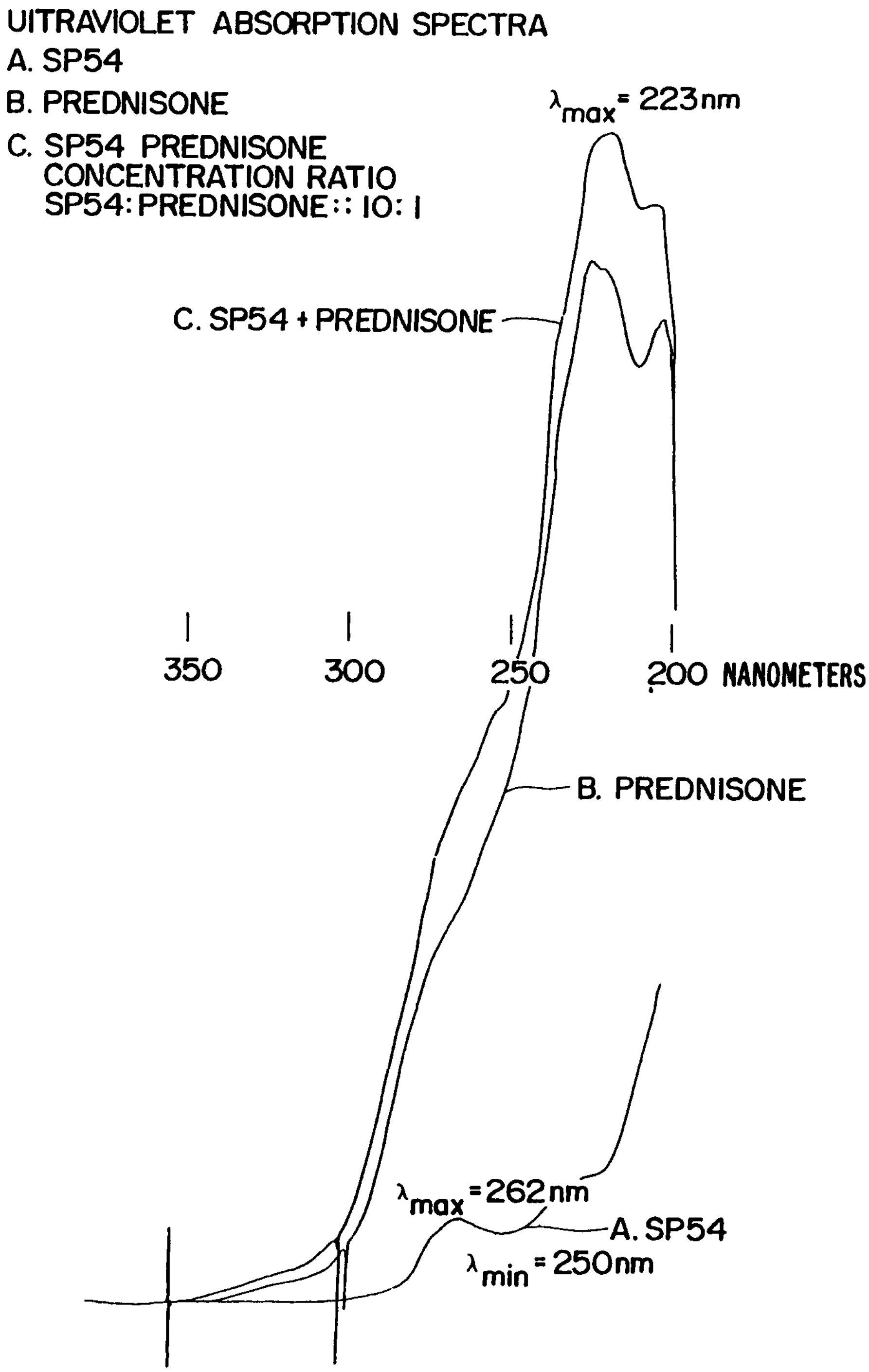
UITRAVIOLET ABSORPTION SPECTRA
A. SP54
B. PREDNISONE
C. SP54 PREDNISONE
CONCENTRATION RATIO
SP54: PREDNISONE :: 10: 1
$\lambda_{max}$ = 223nm
C. SP54 + PREDNISONE
350
300
250
200 NANOMETERS
B. PREDNISONE
$\lambda_{max}$ = 262nm
A. SP54
$\lambda_{min}$ = 250nm

Fig- 4

ULTRAVIOLET SPECTRA OF 0.33% XYLAN SULFATE II (LARCH) ARE SHOWN IN AQUEOUS, pH 5.8, ALKALINE, pH 12.2, AND ACIDIC, pH 1.5 AQUEOUS SOLUTIONS.

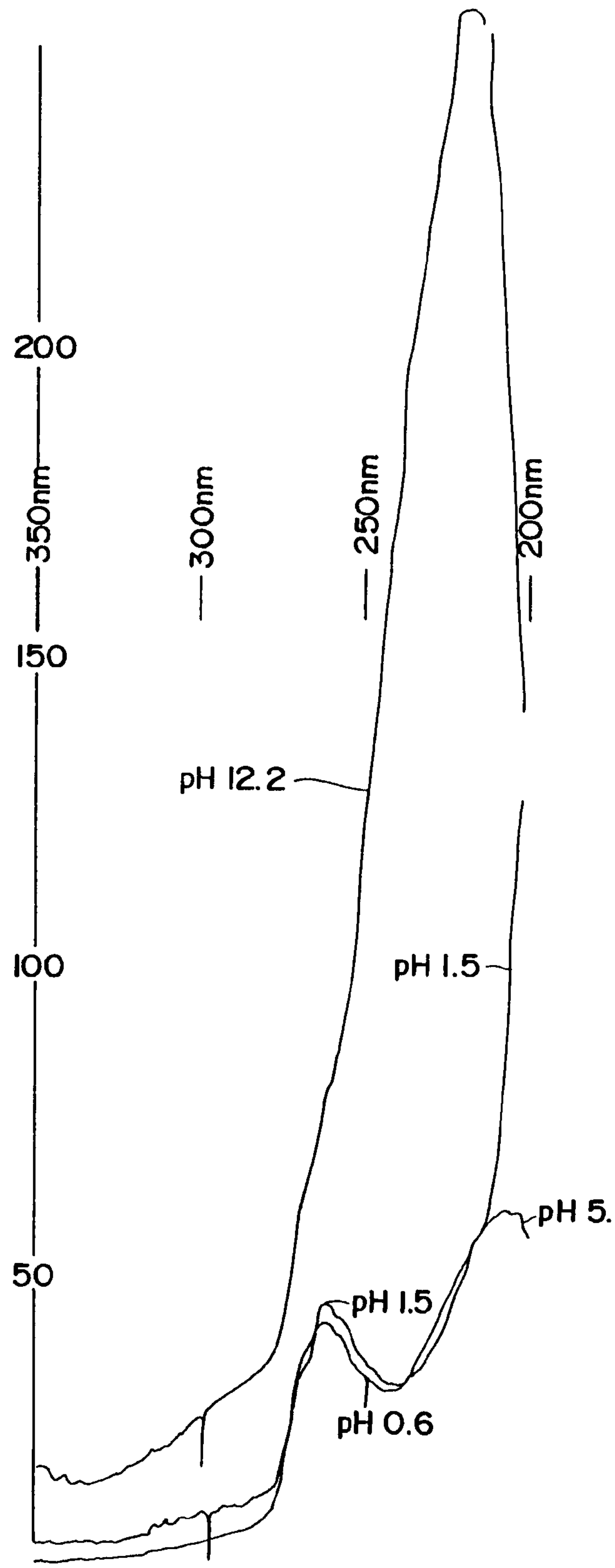


Fig- 5

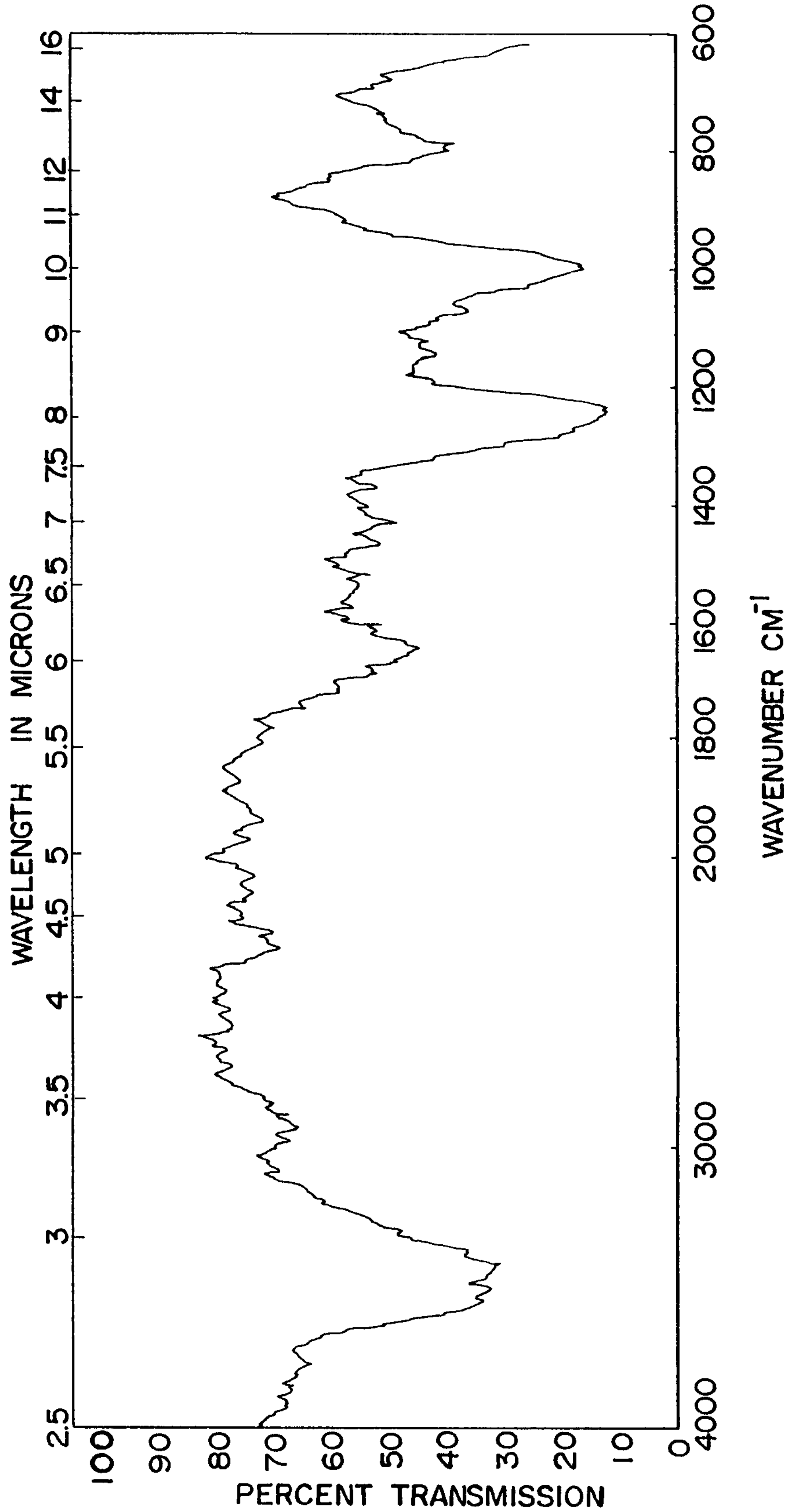
WAVELENGTH IN MICRONS
2.5
3
3.5
4
4.5
5
5.5
6
6.5
7
7.5
8
9
10
11
12
14
16
PERCENT TRANSMISSION
100
90
80
70
60
50
40
30
20
10
0
4000
3000
2000
1800
1600
1400
1200
1000
800
600
WAVENUMBER CM-1

Fig- 6

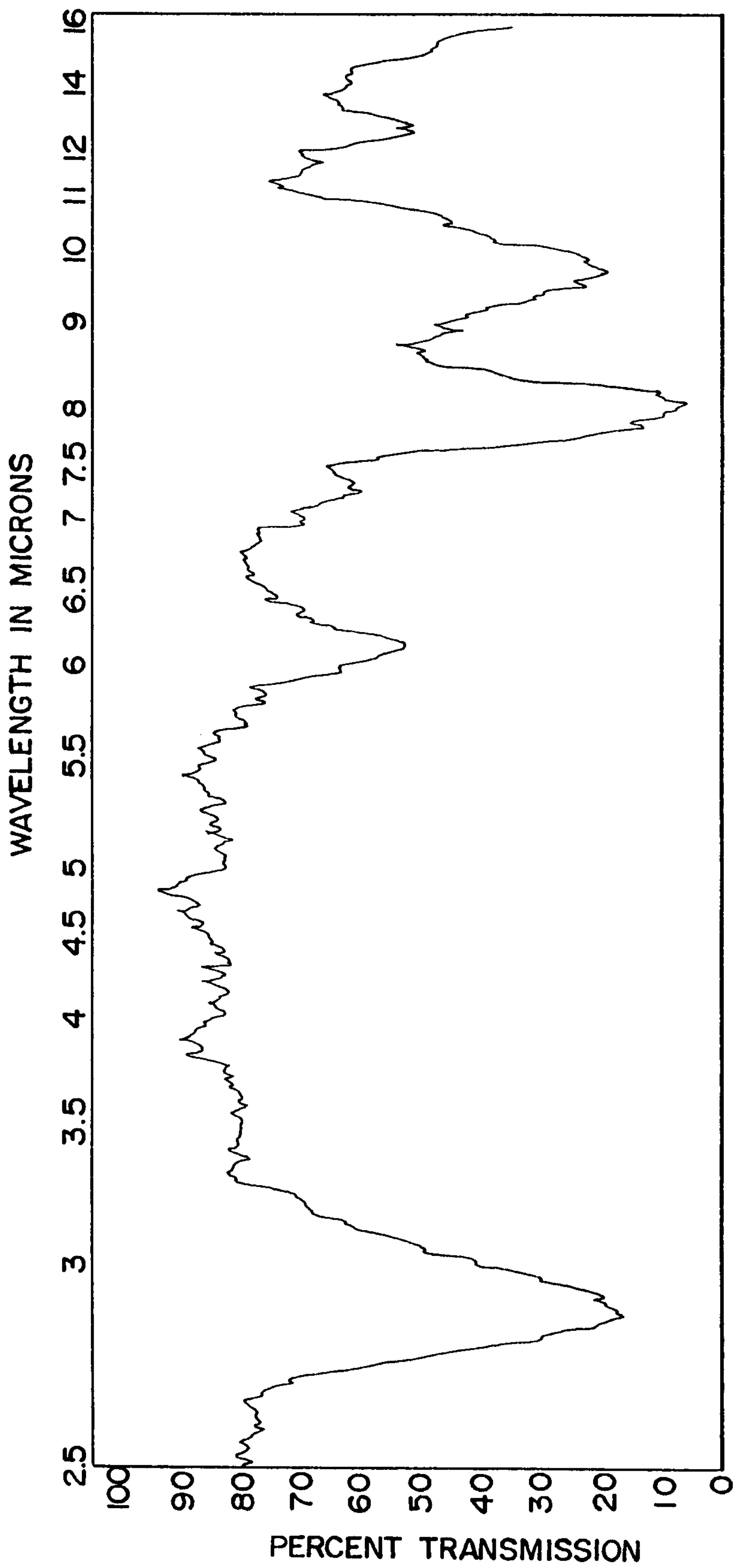
WAVELENGTH IN MICRONS
2.5
3
3.5
4
4.5
5
5.5
6
6.5
7
7.5
8
9
10
11
12
14
16
100
90
80
70
60
50
40
30
20
10
0
PERCENT TRANSMISSION

Fig- 7

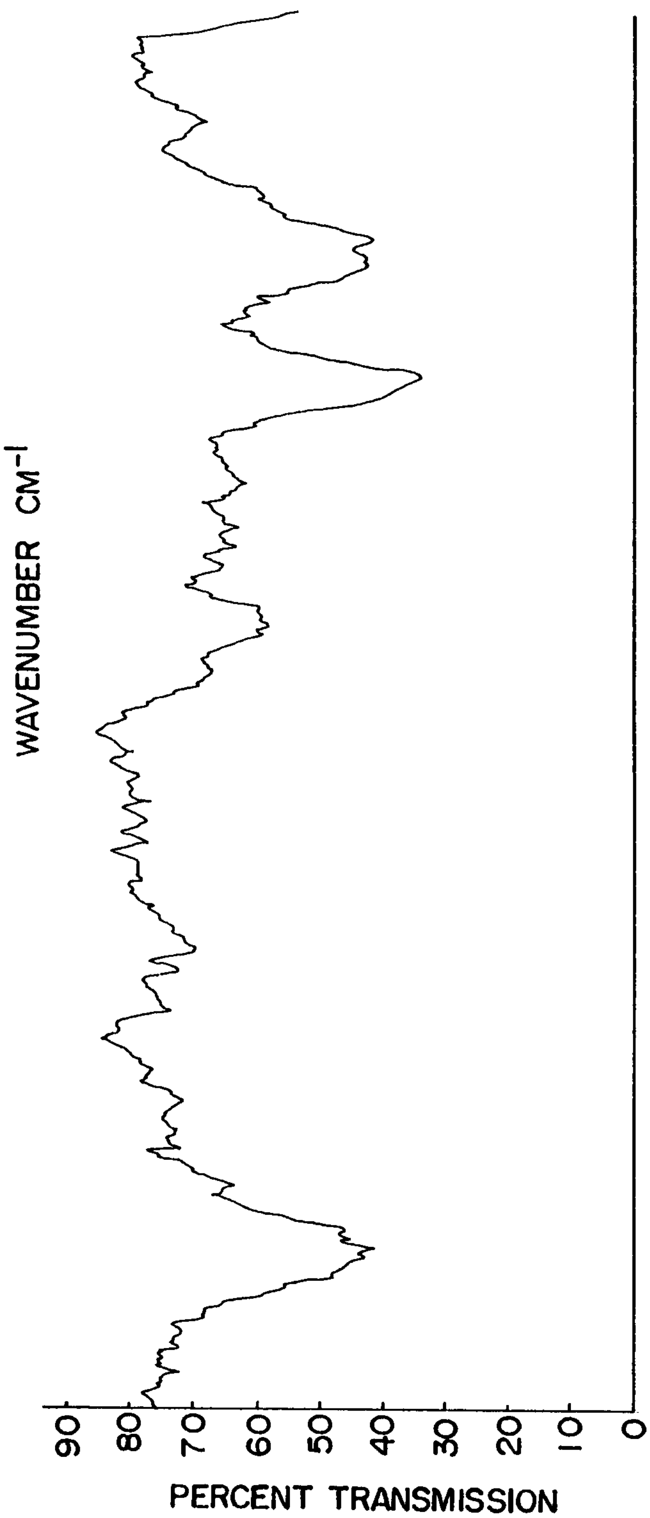
WAVENUMBER CM-1
90 80 70 60 50 40 30 20 10 0
PERCENT TRANSMISSION

Fig- 8

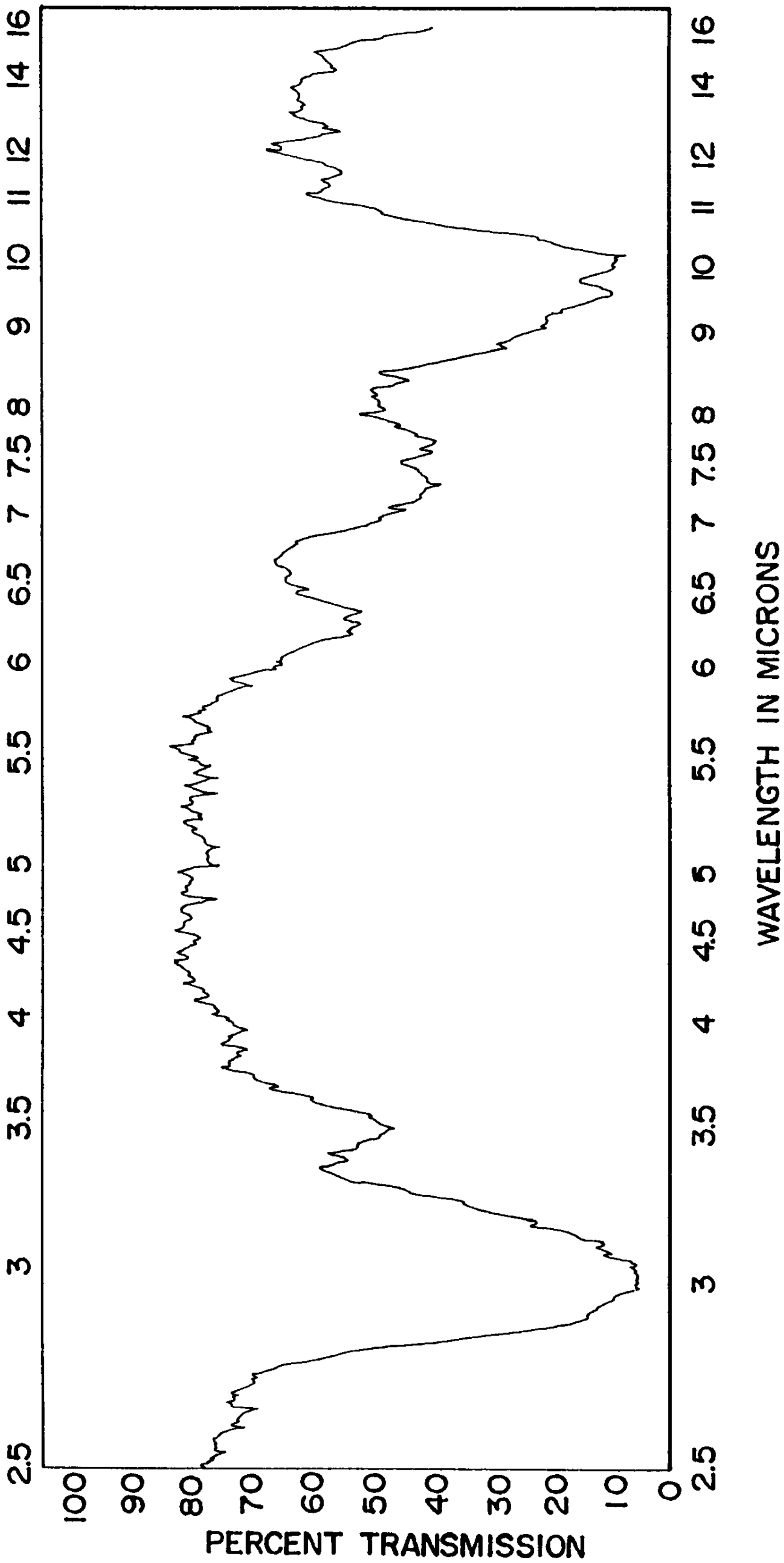
PERCENT TRANSMISSION
100
90
80
70
60
50
40
30
20
10
0
WAVELENGTH IN MICRONS
2.5
3
3.5
4
4.5
5
5.5
6
6.5
7
7.5
8
9
10
11
12
14
16

Fig - 9

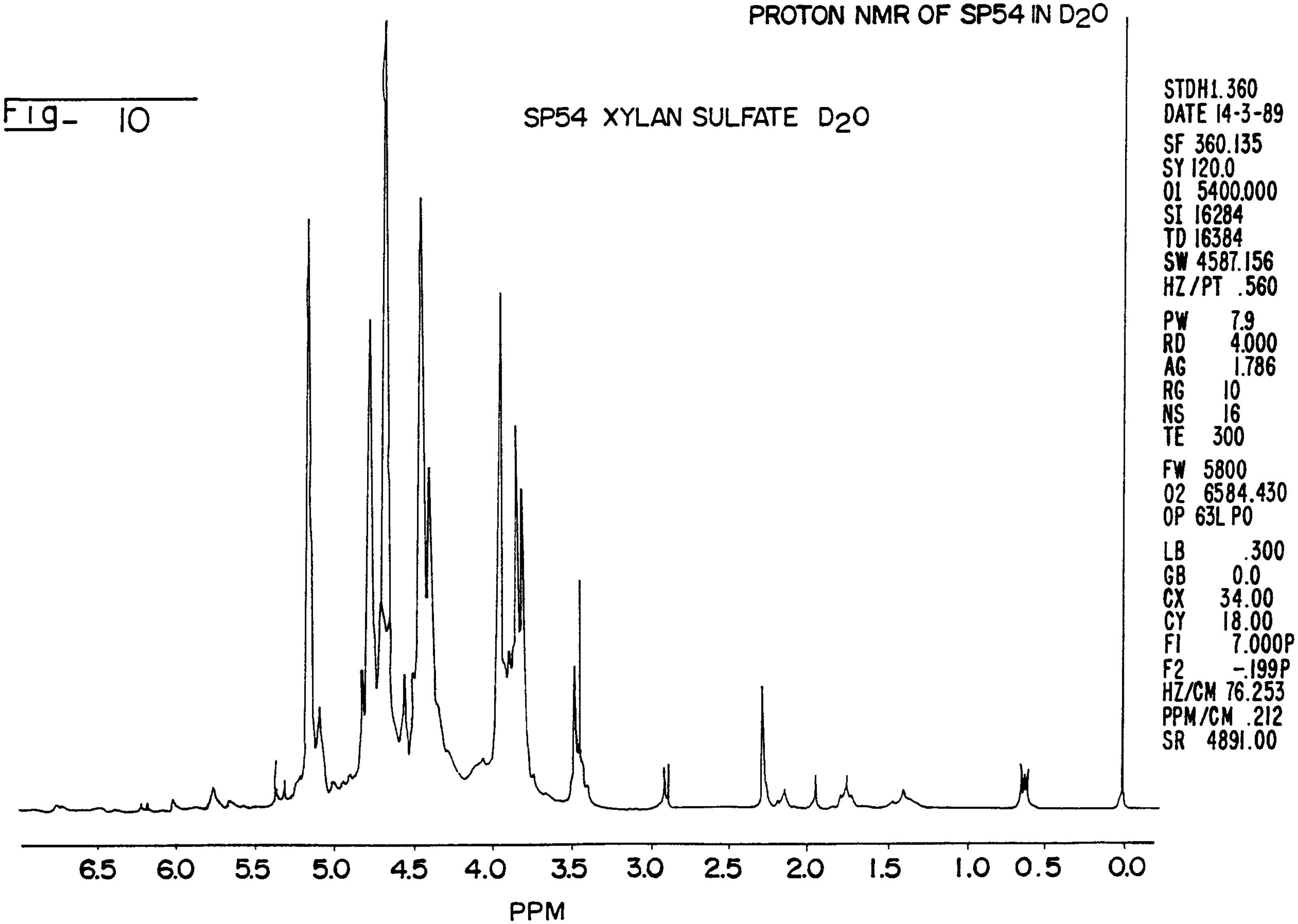
PROTON NMR OF SP54 IN $D_2O$
SP54 XYLAN SULFATE $D_2O$
STDH1.360
DATE 14-3-89
SF 360.135
SY 120.0
01 5400.000
SI 16284
TD 16384
SW 4587.156
HZ/PT .560
PW 7.9
RD 4.000
AG 1.786
RG 10
NS 16
TE 300
FW 5800
02 6584.430
OP 63L PO
LB .300
GB 0.0
CX 34.00
CY 18.00
F1 7.000P
F2 -.199P
HZ/CM 76.253
PPM/CM .212
SR 4891.00
6.5 6.0 5.5 5.0 4.5 4.0 3.5 3.0 2.5 2.0 1.5 1.0 0.5 0.0
PPM

Fig- 10

Fig- 11

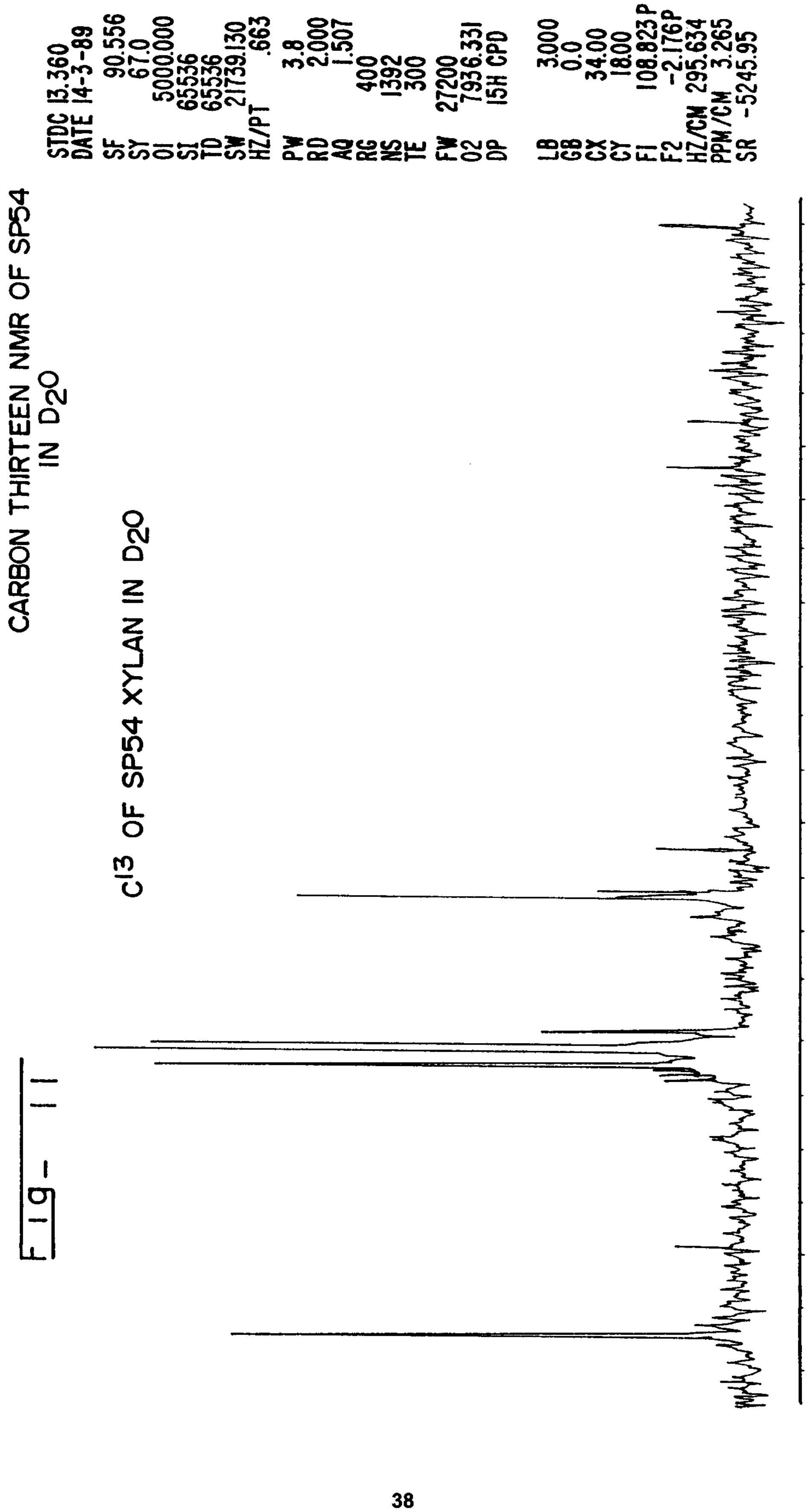
CARBON THIRTEEN NMR OF SP54
IN $D_2O$
$C^{13}$ OF SP54 XYLAN IN $D_2O$
STDC 13.360
DATE 14-3-89
SF 90.556
SY 67.0
O1 5000.000
SI 65536
TD 65536
SW 21739.130
HZ/PT .663
PW 3.8
RD 2.000
AQ 1.507
RG 400
NS 1392
TE 300
FW 27200
O2 7936.331
DP 15H CPD
LB 3.000
GB 0.0
CX 34.00
CY 18.00
F1 108.823P
F2 -2.176P
HZ/CM 295.634
PPM/CM 3.265
SR -5245.95
105 100 95 90 85 80 75 70 65 60 55 50 45 40 35 30 25 20 15 10 5 0
PPM

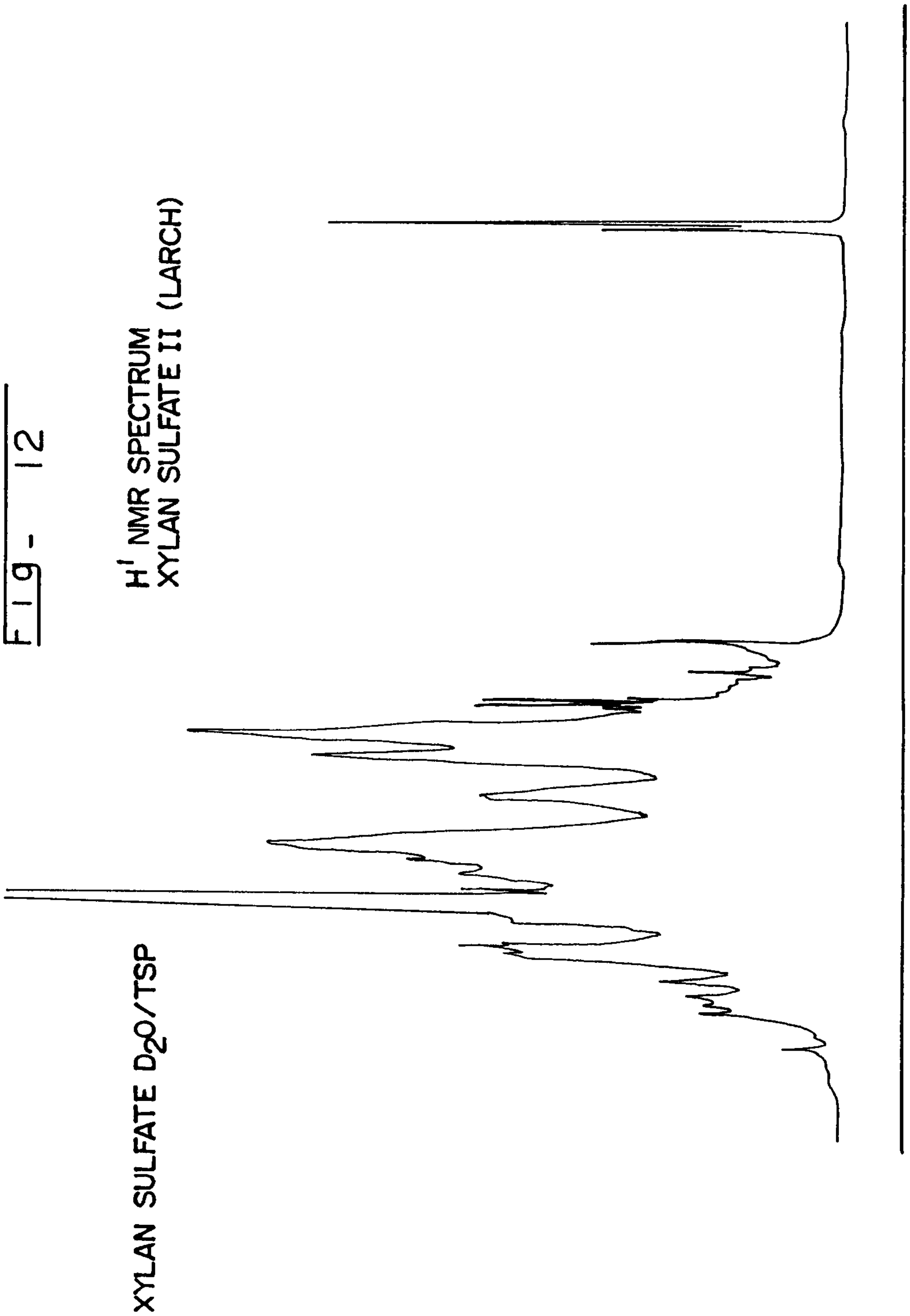


Fig - 12

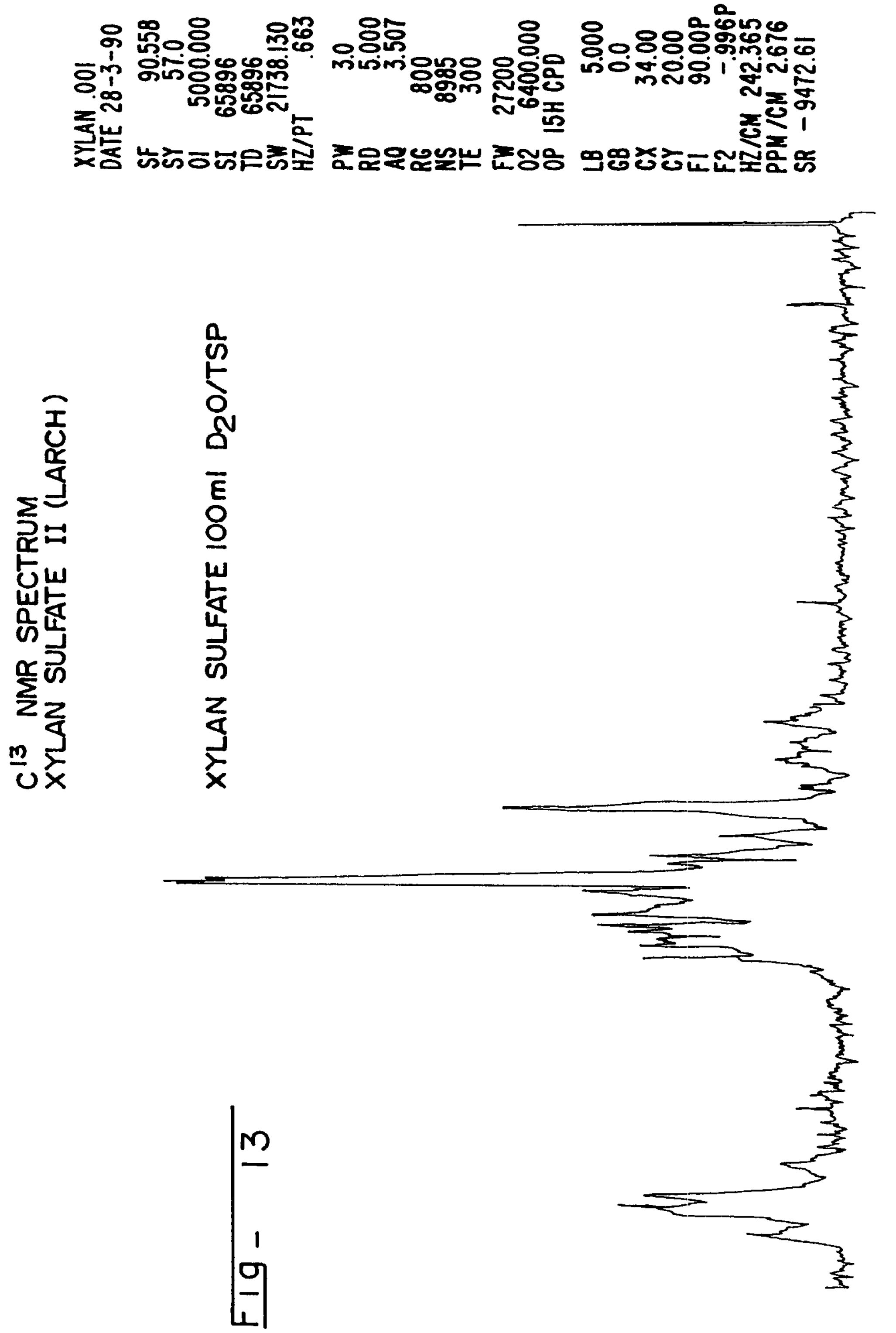
C13 NMR SPECTRUM
XYLAN SULFATE II (LARCH)
XYLAN SULFATE 100ml D2O/TSP
XYLAN .001
DATE 28-3-90
SF 90.558
SY 57.0
O1 5000.000
SI 65896
TD 65896
SW 21738.130
HZ/PT .663
PW 3.0
RD 5.000
AQ 3.507
RG 800
NS 8985
TE 300
FW 27200
O2 6400.000
OP 15H CPD
LB 5.000
GB 0.0
CX 34.00
CY 20.00
F1 90.00P
F2 -.996P
HZ/CM 242.365
PPM/CM 2.676
SR -9472.61
85
80
75
70
65
60
55
50
45
40
35
30
25
20
15
10
5
0
PPM

Fig - 13

SP54 COMMERCIAL 10 %

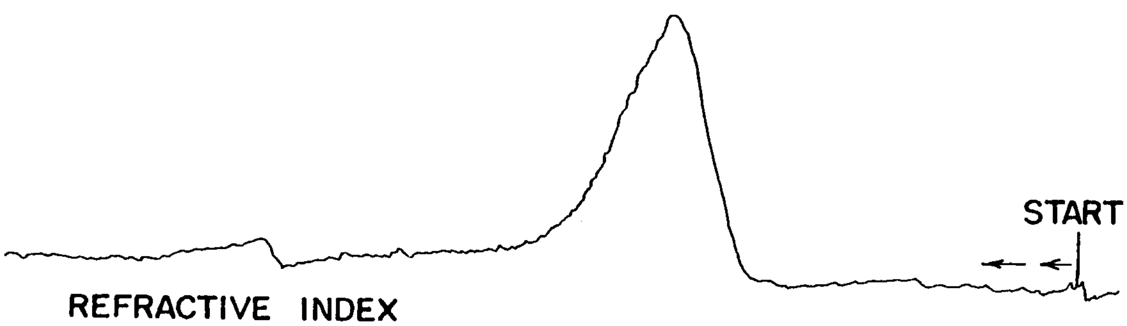


Fig - 14

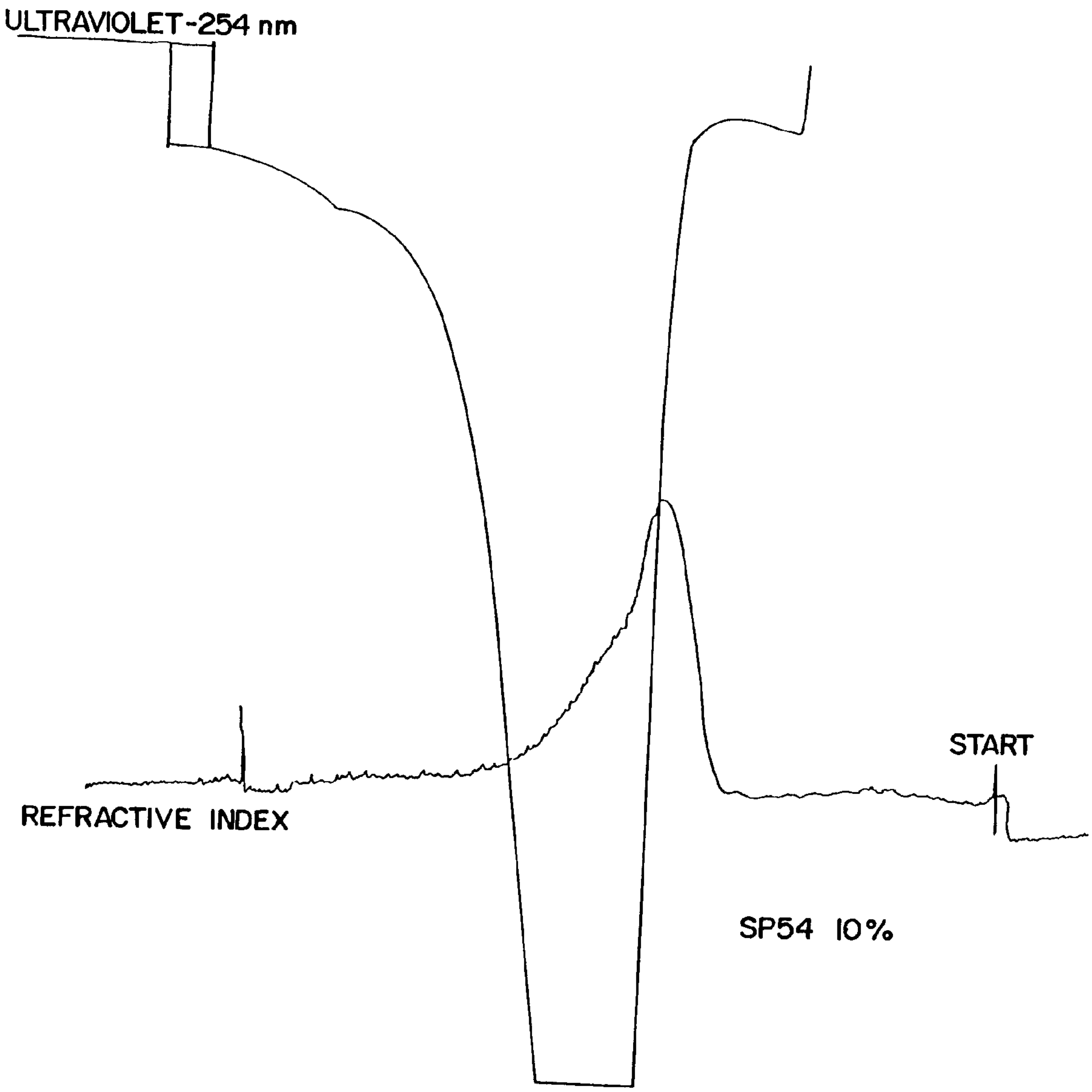


Fig - 15

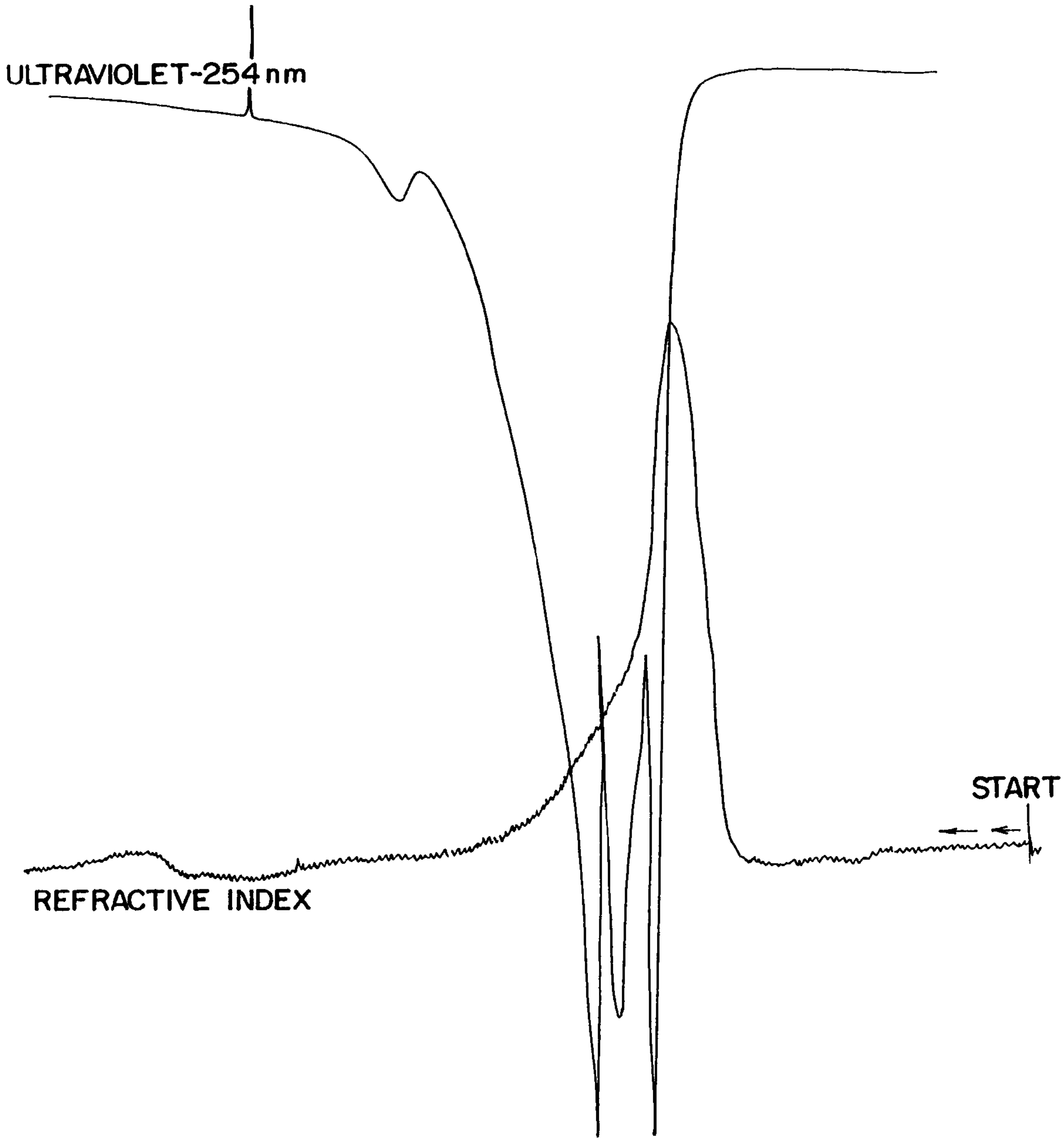


XYLAN SULFATE LARCH I 10%

Fig- 16

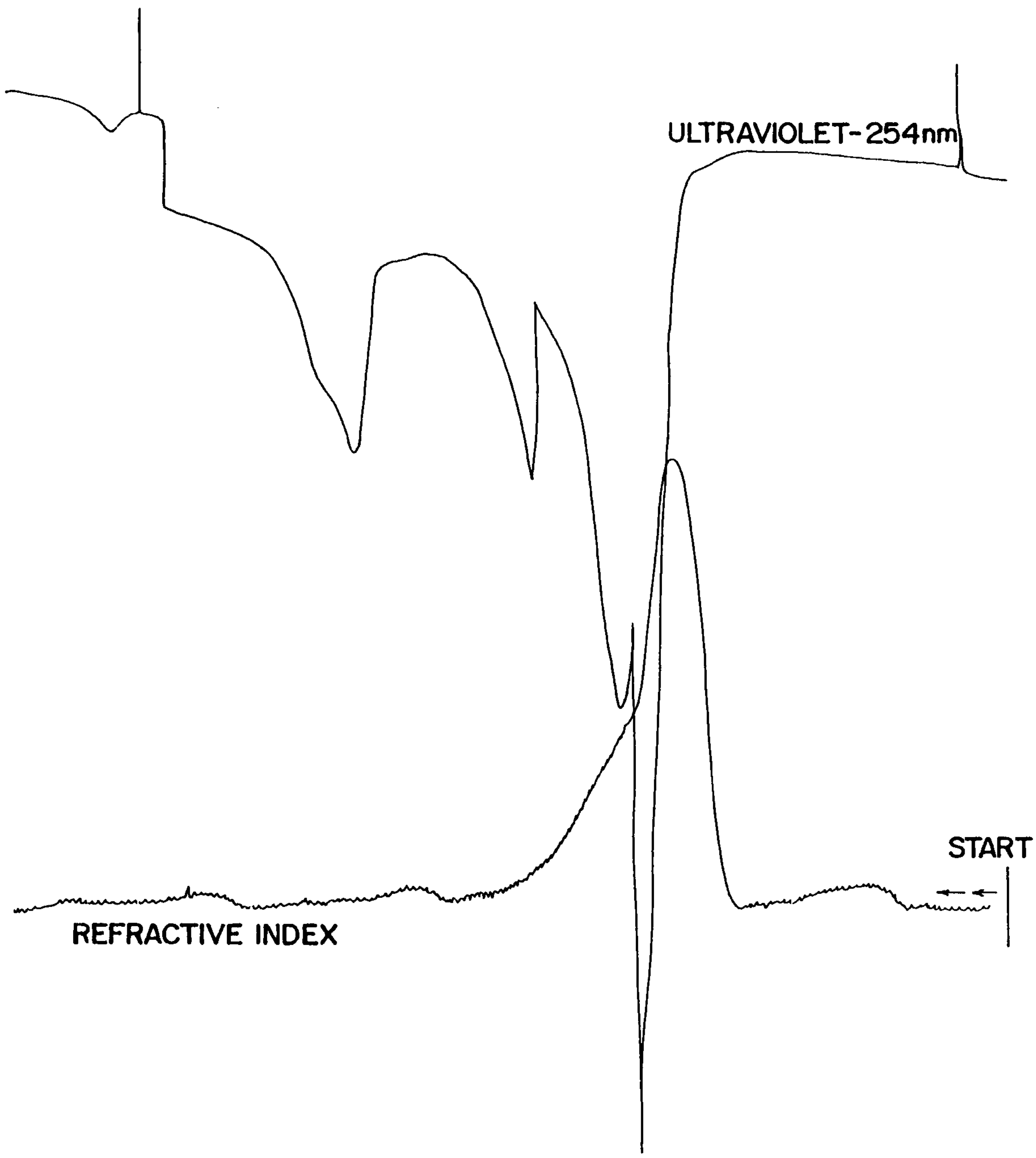
ULTRAVIOLET-254nm
START
REFRACTIVE INDEX
XYLAN SULFATE LARCH II

Fig - 17

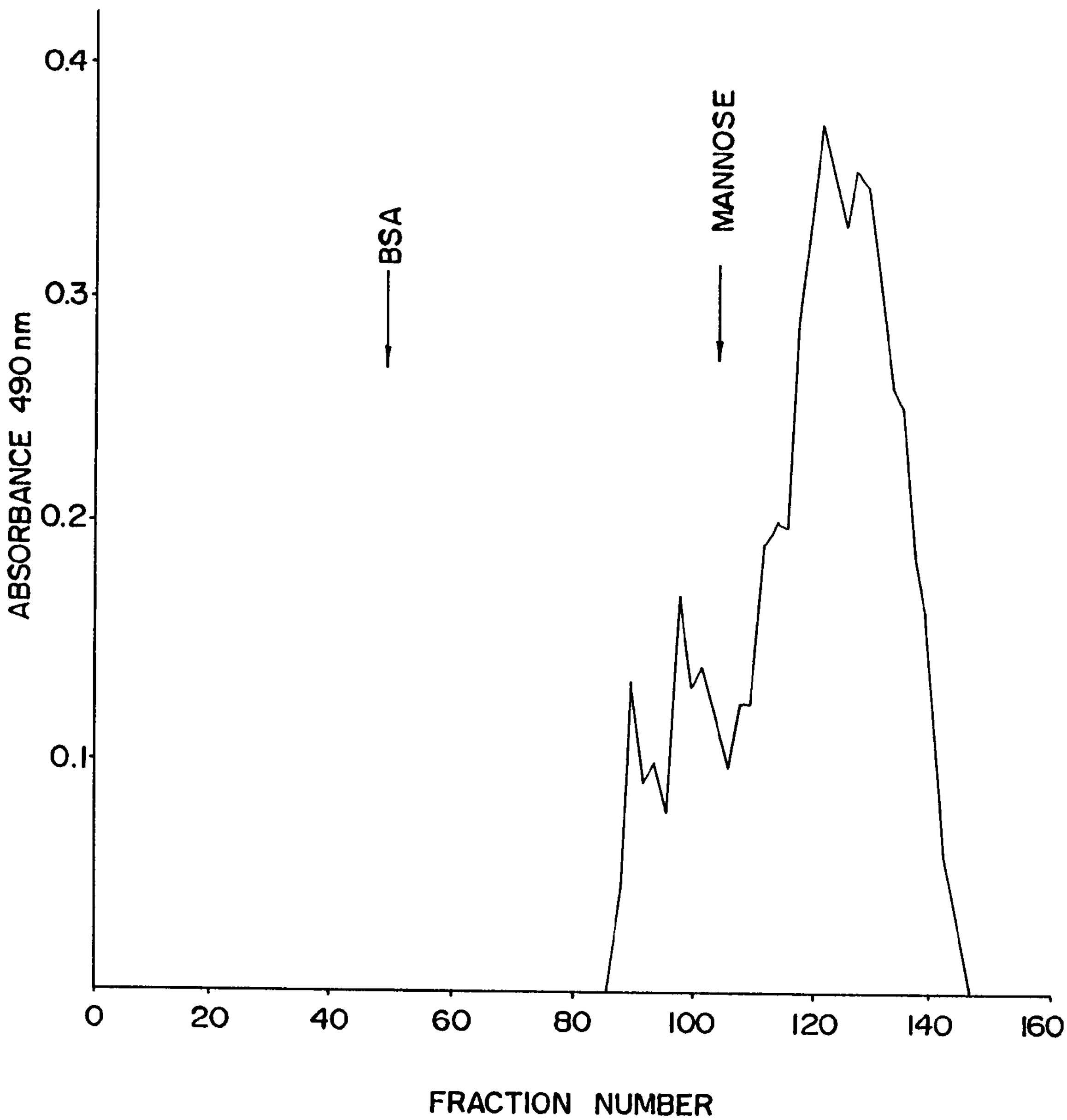
0.4
0.3
0.2
0.1
ABSORBANCE 490 nm
BSA
MANNOSE
0
20
40
60
80
100
120
140
160
FRACTION NUMBER

Fig - 18

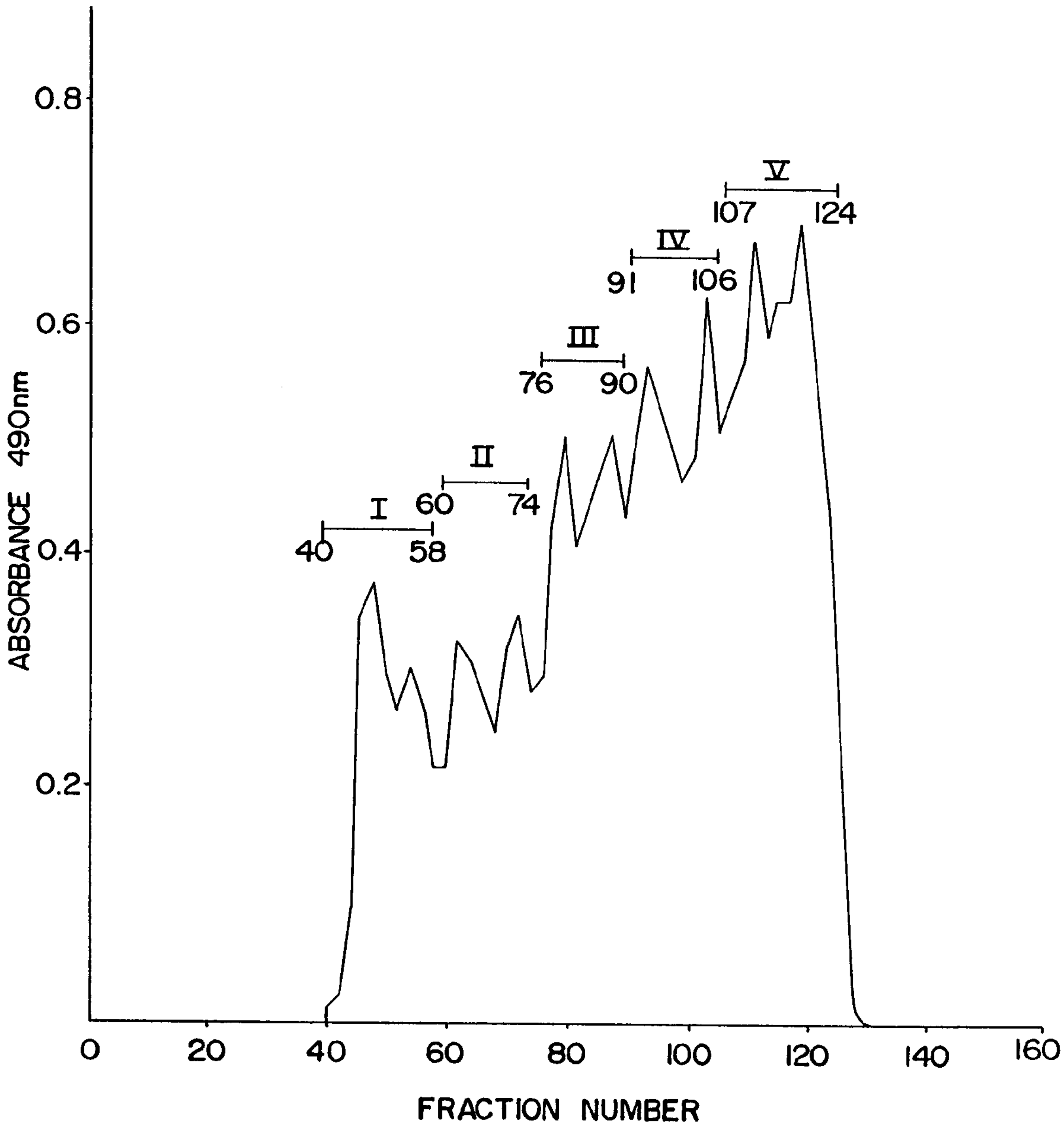
0.8
0.6
0.4
0.2
ABSORBANCE 490nm
I
40
58
II
60
74
III
76
90
IV
91
106
V
107
124
0
20
40
60
80
100
120
140
160
FRACTION NUMBER

Fig - 19

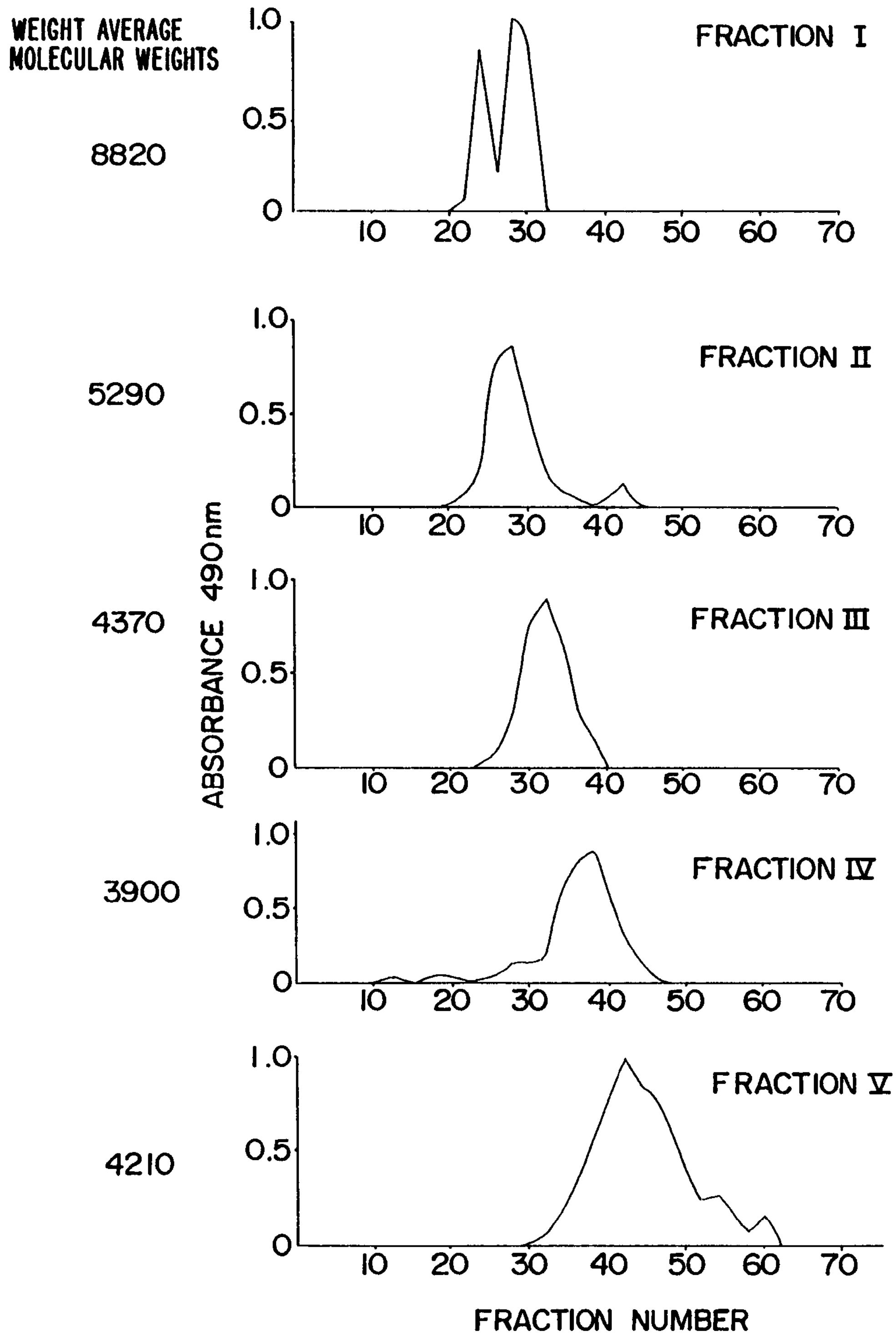
WEIGHT AVERAGE
MOLECULAR WEIGHTS
8820
5290
4370
3900
4210
ABSORBANCE 490nm
1.0
0.5
0
10 20 30 40 50 60 70
FRACTION I
FRACTION II
FRACTION III
FRACTION IV
FRACTION V
FRACTION NUMBER

Fig - 20

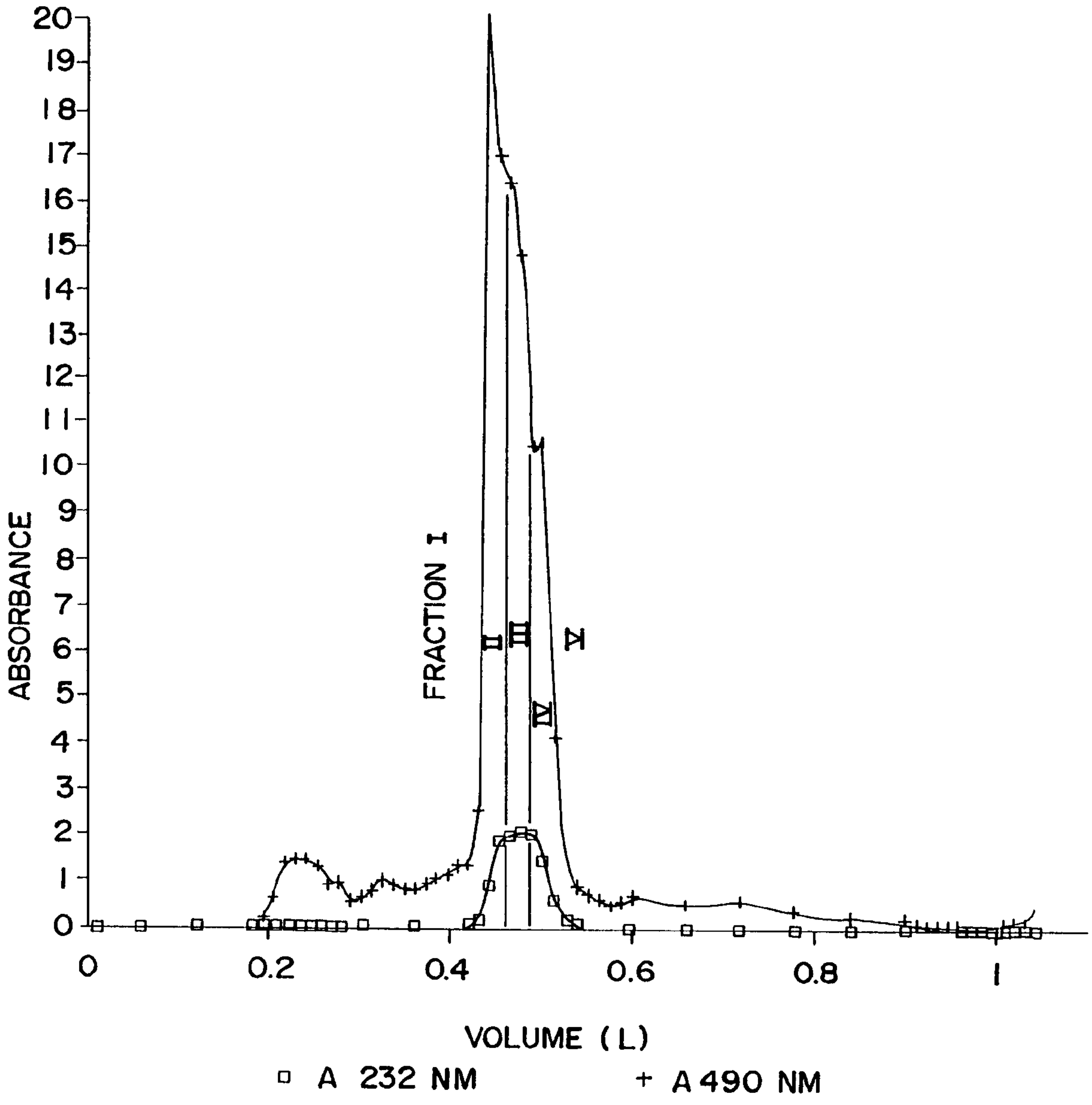
20
19
18
17
16
15
14
13
12
11
10
9
8
7
6
5
4
3
2
1
0
ABSORBANCE
FRACTION I
II
III
IV
V
0
0.2
0.4
0.6
0.8
1
VOLUME (L)
A 232 NM
A 490 NM

Fig - 21

Fig - 22

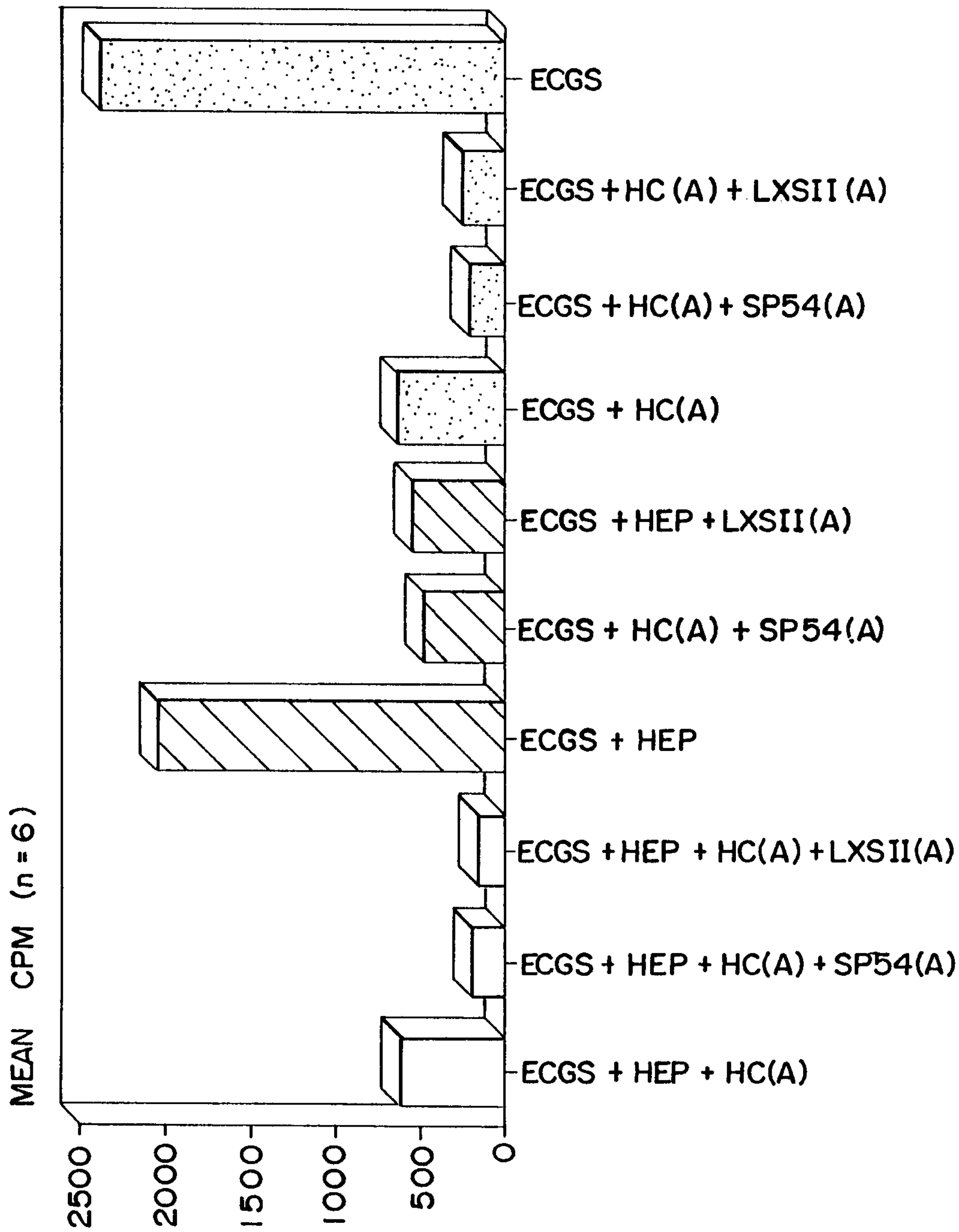
MEAN CPM (n = 6)
2500
2000
1500
1000
500
0
ECGS
ECGS + HC (A) + LXSII (A)
ECGS + HC(A) + SP54(A)
ECGS + HC(A)
ECGS + HEP + LXSII (A)
ECGS + HC(A) + SP54(A)
ECGS + HEP
ECGS + HEP + HC(A) + LXSII(A)
ECGS + HEP + HC(A) + SP54(A)
ECGS + HEP + HC(A)

Fig - 23

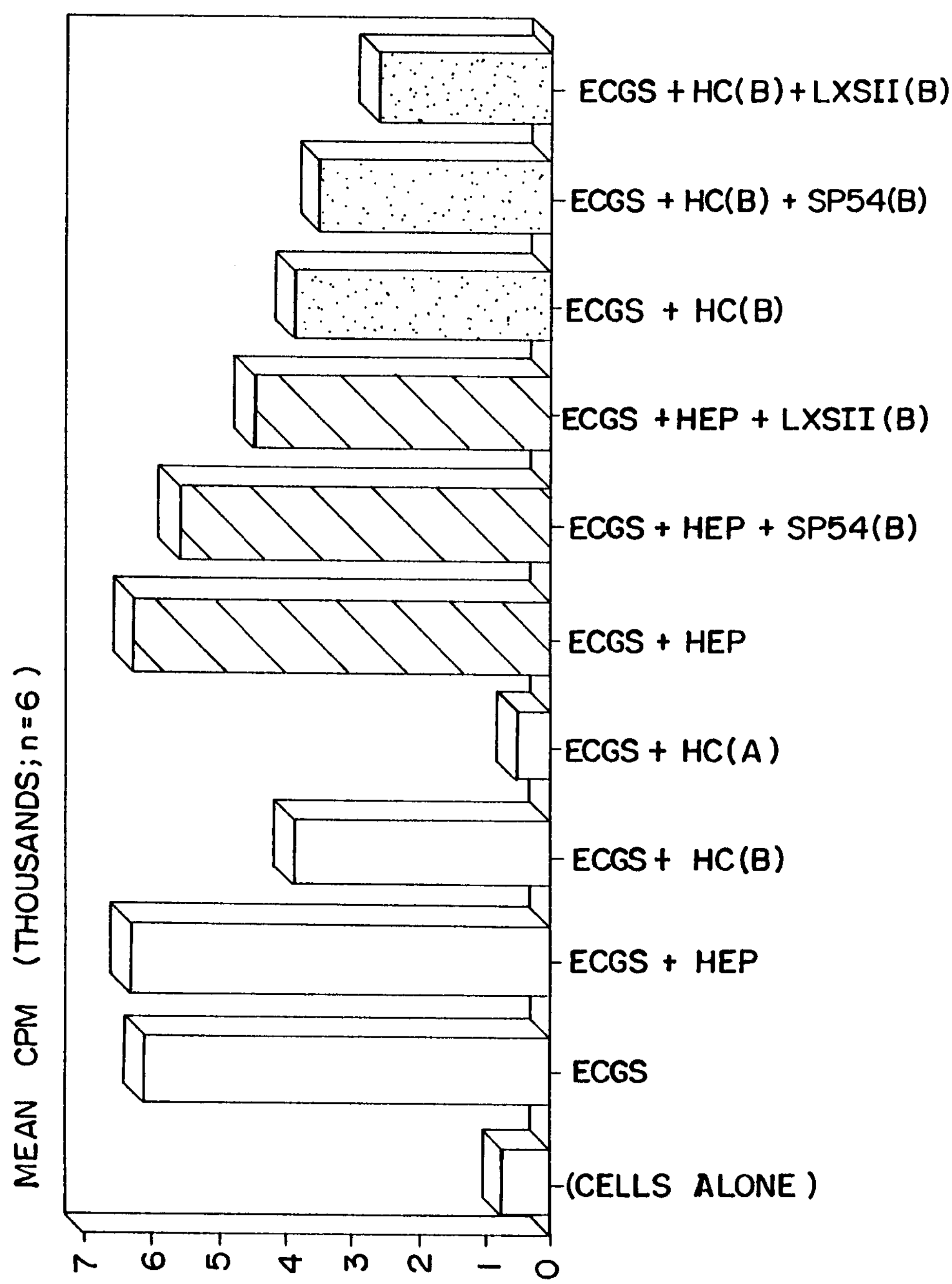
MEAN CPM (THOUSANDS; n=6)
7
6
5
4
3
2
1
0
(CELLS ALONE)
ECGS
ECGS + HEP
ECGS + HC(B)
ECGS + HC(A)
ECGS + HEP
ECGS + HEP + SP54(B)
ECGS + HEP + LXSII (B)
ECGS + HC(B)
ECGS + HC(B) + SP54(B)
ECGS + HC(B) + LXSII (B)